(19) Europäisches Patentamt
European Patent Office
Office européen des brevets

(11) EP 4 021 405 B1

(12) **EUROPEAN PATENT SPECIFICATION**

(45) Date of publication and mention of the grant of the patent:
**06.11.2024 Bulletin 2024/45**

(21) Application number: **20764963.3**

(22) Date of filing: **27.08.2020**

(51) International Patent Classification (IPC):
***A61K 9/00*** (2006.01) ***A61K 47/02*** (2006.01)
***A61K 47/26*** (2006.01) ***A61K 47/32*** (2006.01)
***A61K 47/36*** (2006.01) ***A61K 47/38*** (2006.01)
***A61K 47/40*** (2006.01) ***A61K 31/135*** (2006.01)

(52) Cooperative Patent Classification (CPC):
**A61K 9/006; A61K 31/135; A61K 47/02; A61K 47/26; A61K 47/32; A61K 47/36; A61K 47/38; A61K 47/40**

(86) International application number:
**PCT/EP2020/073940**

(87) International publication number:
**WO 2021/037960 (04.03.2021 Gazette 2021/09)**

(54) **PHARMACEUTICAL FORMULATION**

PHARMAZEUTISCHE FORMULIERUNG

FORMULATION PHARMACEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2019 GB 201912505**

(43) Date of publication of application:
**06.07.2022 Bulletin 2022/27**

(73) Proprietor: **Klaria Pharma Holding AB**
**754 50 Uppsala (SE)**

(72) Inventors:
- **BOYER, Scott**
  **754 50 Uppsala (SE)**
- **HÜBINETTE, Fredrik**
  **754 50 Uppsala (SE)**

(74) Representative: **J A Kemp LLP**
**80 Turnmill Street**
**London EC1M 5QU (GB)**

(56) References cited:
**WO-A1-2017/003935** **WO-A1-2018/224674**
**US-A1- 2015 224 070**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to films comprising an alginate salt of a monovalent cation or a mixture of alginate salts containing at least one alginate salt of a monovalent cation, and a compound of Formula (I), such as ketamine, or a pharmaceutically acceptable salt thereof. The present invention further relates to methods for manufacturing such films, and such films for use in anesthesia, pain management, and the treatment of disease, in particular amnesia, depression and bipolar disorder.

### Background to the Invention

Ketamine is an active agent used in anesthesia, pain management, and the treatment of amnesia, depression and bipolar disorder. In addition, ketamine can induce bronchodilation and avoid cardiovascular depression [1]. In particular, it is used as short-acting anesthetic agent in humans and in some animal species. However, it is used as an analgesic in subanesthetic doses where it acts as non-competitive N-methyl-D-aspartate (NMDA) receptor antagonist [2].

Chemically, ketamine is an arylcyclohexylamine derivative that has chiral forms. In general, most pharmaceutical formulations of ketamine are a racemic mixture. However, the pharmacologically more active enantiomer esketamine (S-ketamine) is also commercially available for medical use under brand name "Ketanest S" while the less active enantiomer arketamine (R-ketamine) has not been available for clinical use. Optical rotation of an enantiomer of ketamine can vary between its salts and free base form due to change in conformation of cyclohexanone ring. For example, free base (S)-ketamine is dextrorotatory and labelled as S-(+)-ketamine whereas its hydrochloride salt exhibits levorotation and is thus labelled S-(-)-ketamine hydrochloride.

Ketamine has a strongest basic $pK_a$ of 7.6 and a logP value of 3.35. In solution, ketamine is most stable at a pH around 4 in its ionized form. *Ketalar* intravenous injections, available on the market, are formulated at a slightly acidic pH (3.5-5.5) and are effective as compared to what would be expected based on the knowledge of the $pK_a$. However, intravenous injection is invasive and carries a risk of needle-stick injuries in first responders and caregivers. Intravenous injections may also lead to overdose. Oral formulations of ketamine are also available, but result in low bioavailability (typically around 40-45%) as ketamine undergoes first pass metabolism, where it is biotransformed in the liver by CYP3A4, CYP2B6 and CYP2C9 isoenzymes into norketamine (through N-demethylation) and finally dehydronorketamine [3]. Nasal sprays of ketamine are also available, but routinely lead to an inconsistent dose of ketamine being administered to the recipient, with bioavailability varying from 8 to 50%.

In summary, no formulation of ketamine is currently available which can be administered in a non-invasive fashion, is needle-free and which results in acceptable bioavailability and blood plasma concentrations of ketamine with low variability between patients.

Other aryl cyclohexylamines and analogues of ketamine also useful in medicine include eticylidine (PCE), 3-methoxyeticyclidine, methoxetamine (MXE), tiletamine, phencyclidine (PCP), tenocylidine (TCP) and many others.

US 2015/224070 describes a gelling formulation containing ketamine or a pharmaceutically acceptable salt thereof in water, a poloxamer and one or more pharmaceutically acceptable mucoadhesive agents.

### Summary of the Invention

The present invention is based on the unexpected finding that formulations of a compound of Formula (I), such as ketamine, or pharmaceutically acceptable salts thereof, in a film suitable for administration to an oral cavity can provide an advantageous balance of properties. This balance of properties is desirable for use in anesthesia, pain management, and the treatment of amnesia, depression and bipolar disorder. In particular, film formulations of ketamine can potentially provide a needle-free alternative to intravenous formulations, whilst enabling acceptable plasma levels of ketamine to be delivered to patients, with low variability between patients. This makes the present ketamine-containing film formulations more attractive for use than existing oral and intranasal formulations.

Hence, the invention provides for the first time a film suitable for administration to an oral cavity comprising a compound of Formula (I), such as ketamine, its use in anesthesia, pain management, and the treatment of amnesia, depression and bipolar disorder, and methods for its manufacture.

In one aspect, the present invention provides a film suitable for administration to an oral cavity comprising:

(i) an alginate salt of a monovalent cation or a mixture of alginate salts containing at least one alginate salt of a monovalent cation;
(ii) an active pharmaceutical ingredient (API) which is a compound of Formula (I)

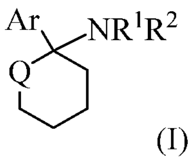

(I)

wherein:

Ar is selected from

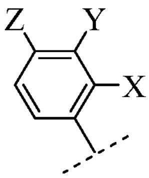 and 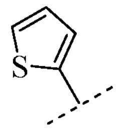 ;

X is selected from hydrogen, halo, OH, $NH_2$, methyl, trifluoromethyl and methoxy;
Y is selected from hydrogen, halo, OH, $NH_2$, methyl, trifluoromethyl and methoxy;
Z is selected from hydrogen, halo, OH, $NH_2$, methyl, trifluoromethyl and methoxy;
Q is selected from $-CH_2-$, -CH(OH)-, -CH(Me)-, -CH(OMe)-, -(C=O)-, -(C=S)- and -(C=NR)-, wherein R is selected from hydrogen or $C_{1-6}$ alkyl;
$R^1$ is selected from hydrogen and $C_{1-6}$ alkyl and $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl, or $R^1$ and $R^2$ are linked so as to form a bivalent alkylene moiety having from 3 to 7 carbon atoms; and

(iii) an acid $H_xA$, wherein A is a counterion having an ionic radius of 2.70 or greater, and x is a positive integer which is equal to the charge on the counterion A;

further wherein the alginate salt of a monovalent cation (a) comprises from 25 to 35% by weight of β-D-mannuronate and/or from 65 to 75% by weight of α-L-guluronate, and (b) has a weight average molecular weight of from 30,000 g/mol to 90,000 g/mol.

In another aspect, the present invention provides a film suitable for administration to an oral cavity comprising:

(i) an alginate salt of a monovalent cation or a mixture of alginate salts containing at least one alginate salt of a monovalent cation;
(ii) an active pharmaceutical ingredient (API) which is a pharmaceutically acceptable salt of a compound of Formula (I)

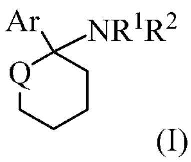

(I)

wherein:

Ar is selected from

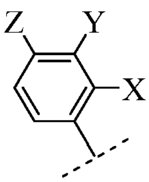 and 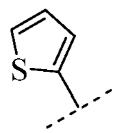 ;

X is selected from hydrogen, halo, OH, $NH_2$, methyl, trifluoromethyl and methoxy;
Y is selected from hydrogen, halo, OH, $NH_2$, methyl, trifluoromethyl and methoxy;
Z is selected from hydrogen, halo, OH, $NH_2$, methyl, trifluoromethyl and methoxy;
Q is selected from $-CH_2-$, -CH(OH)-, -CH(Me)-, -CH(OMe)-, -(C=O)-, -(C=S)- and -(C=NR)-, wherein R is selected from hydrogen or $C_{1-6}$ alkyl;
$R^1$ is selected from hydrogen and $C_{1-6}$ alkyl and $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl, or $R^1$ and $R^2$ are linked so as to form a bivalent alkylene moiety having from 3 to 7 carbon atoms; and

(iii) an additive selected from xylitol, a cyclodextrin, poly(vinyl pyrrolidone), hydroxypropylmethylcellulose, poly(acrylic acid) and pullulan;

further wherein the alginate salt of a monovalent cation (a) comprises from 25 to 35% by weight of β-D-mannuronate and/or from 65 to 75% by weight of α-L-guluronate, and (b) has a weight average molecular weight of from 30,000 g/mol to 90,000 g/mol.

In another aspect, the present invention provides a film according to the invention for use in the treatment of a human patient.

In another aspect, the present invention provides a film according to the invention for use in anesthesia, pain management, or the treatment of a condition selected from amnesia, depression and bipolar disorder, in a human patient.

In another aspect, the present invention provides a method of manufacturing a film according to the invention, said method comprising the following steps:

(a) mixing the API in water, and optionally subsequently adjusting the pH of the solution to the desired level by addition of an appropriate acid or base, typically a concentrated acid, and preferably adjusting the pH of the solution to from 2 to 4;
(b) optionally, mixing one or more excipients into the solution;
(c) adding the alginate salt of monovalent cation under suitable conditions to result in the formation of a viscous cast;
(d) adjusting the pH of the solution to the desired level by addition of an appropriate acid or base, typically a diluted acid or alkali, preferably a diluted alkali, and preferably adjusting the pH of the solution to from 3 to 5;
(e) optionally, sonicating the cast;
(f) leaving the cast to de-aerate;
(g) pouring the cast onto a surface and spreading the cast out to the desired thickness;
(h) drying the cast layer, typically at a temperature of from 30 to 70 °C, until the residual water content of the film is from 0 to 20% by weight and a solid film is formed; and
(i) optionally, cutting the solid film into pieces of the desired size, further optionally placing these pieces into pouches, preferably wherein the pouches are made from PET-lined aluminium, sealing the pouches and further optionally, labelling them.

In another aspect, the present invention provides a method of manufacturing a film according to the invention, said method comprising the following steps:

(a) mixing the salt of the API in water;
(b) adding one or more additives selected from xylitol, a cyclodextrin, poly(vinyl pyrrolidone), hydroxypropylmethylcellulose, poly(acrylic acid) and pullulan to the solution;
(c) optionally, mixing one or more excipients into the solution;
(d) adding the alginate salt of monovalent cation under suitable conditions to result in the formation of a viscous cast;
(e) optionally, adding further water to the cast;
(f) optionally, sonicating the cast;
(g) leaving the cast to de-aerate;
(h) pouring the cast onto a surface and spreading the cast out to the desired thickness;
(i) drying the cast layer, typically at a temperature of from 30 to 70 °C, until the residual water content of the film is from 0 to 20% by weight and a solid film is formed; and
(j) optionally, cutting the solid film into pieces of the desired size, further optionally placing these pieces into pouches, preferably wherein the pouches are made from PET-lined aluminium, sealing the pouches and further optionally, labelling them.

## Brief Description of the Figures

Fig. 1 shows the dissolution pattern of a ketamine 5 mg film: fresh versus old. Dissolution time (DT) of placebo film was considered as control. DT = dissolution time.

Fig. 2 shows dose-adjusted plasma levels of ketamine in the blood plasma of adult beagle dogs (n=3) over a time period of 0 to 480 minutes after administration of a 5 mg single ketamine film (F1), a 10 mg single ketamine film (F2), two 5 mg ketamine films (F3) or a 5 mg intravenous injection of ketamine (F4). All dose levels were adjusted to 10 mg dose equivalents.

Fig. 3 shows dose-adjusted plasma levels of ketamine in the blood plasma of adult beagle dogs (n=3) over a time period of 0 to 60 minutes after administration of a 5 mg single ketamine film (F 1), a 10 mg single ketamine film (F2), two 5 mg ketamine films (F3) or a 5 mg intravenous injection of ketamine (F4). All dose levels were adjusted to 10 mg dose equivalents.

## Detailed Description of the Invention

The present invention is concerned with a film, suitable for administration to an oral cavity, which can be used for delivery of a compound of Formula (I), such as ketamine, or a pharmaceutically acceptable salt thereof to a human patient. Such a film may also be referred to as an oral dissolvable film (ODF) and/or an oral transmucosal film (OTF). The film is typically an alginate film which is applied by the patient themselves or another person, e.g. a medical practitioner, a nurse, a carer, a social worker, a colleague of the patient or a family member of the patient, to the mucosa of the oral cavity. The film is bioadhesive and adheres to the surface of the oral cavity upon application. After application, the alginate film begins to dissolve, releasing the active pharmaceutical ingredient. The present invention is useful in particular in anesthesia, pain management, and the treatment of amnesia, depression and bipolar disorder.

For the avoidance of doubt, all alternative and preferred features relating to the film *per se* apply equally to the use of said film in the treatment of a human patient.

*Definitions*

As defined herein, the term "alkyl" refers to a linear or branched saturated monovalent hydrocarbon radical having the number of carbon atoms indicated in the prefix. Thus, the term "$C_{1-6}$ alkyl" refers to a linear saturated monovalent hydrocarbon radical of one to six carbon atoms or a branched saturated monovalent hydrocarbon radical of three or to six carbon atoms, e.g. methyl, ethyl, *n*-propyl, *iso*-propyl, *n*-butyl, *iso*-butyl, *tert*-butyl and the like. Preferably an alkyl group is a $C_{1-6}$ alkyl group, and more preferably a $C_{1-4}$ alkyl group.

As defined herein, the term "acyl" refers to a -COR radical, wherein R is alkyl, haloalkyl, cycloalkyl, cycloalkylalkyl, aryl, aralkyl, heteroaryl, heteroaralkyl, heterocyclyl, or heterocyclylalkyl, each as defined herein, or poly(ethylene glycol), and wherein R is optionally further substituted with one, two, three, four or more substituents independently selected from alkyl, alkoxy, halo, haloalkoxy, -OH, $-NH_2$, alkylamino or -COOH.

As defined herein, the term "alkylene" refers to a linear saturated divalent hydrocarbon radical or a branched saturated divalent hydrocarbon radical having the number of carbon atoms indicated in the prefix, e.g. methylene, ethylene, propylene, 1-methylpropylene, 2-methylpropylene, butylene, pentylene, and the like. Preferably, an alkylene group is a $C_{1-8}$ alkylene group, and more preferably a $C_{3-6}$ alkylene group.

As used herein, the term "alkenyl" refers to a linear or branched saturated monovalent hydrocarbon radical having the number of carbon atoms indicated in the prefix and containing at least one double bond. Thus, the term "$C_{2-6}$ alkenyl" refers to a linear saturated monovalent hydrocarbon radical of two to six carbon atoms having at least one double bond, or a branched saturated monovalent hydrocarbon radical of three to six carbon atoms having at least one double bond, e.g. ethenyl, propenyl, 1,3-butadienyl, $(CH_2)_2CH=C(CH_3)_2$, $CH_2CH=CHCH(CH_3)_2$, and the like. Preferably, an alkenyl group is a $C_{2-6}$ alkenyl group, and more preferably a $C_{2-4}$ alkenyl group.

As defined herein, the term "alkoxy" refers to an -OR radical where R is alkyl as defined above, e.g., methoxy, ethoxy, *n*-propoxy, *iso*-propoxy, *n*-butyl, *iso*-butyl*, tert*-butyl and the like. Preferably an alkoxy group is a $C_{1-6}$ alkoxy group, and more preferably a $C_{1-4}$ alkoxy group.

As defined herein, the term "alkoxycarbonyl" refers to a -C(O)OR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, or poly(ethylene glycol), and wherein R is optionally further substituted with one, two, three, four or more substituents independently selected from alkyl, alkoxy, halo, haloalkoxy, -OH, $-NH_2$, alkylamino or -COOH.

As defined herein, the term "alkylamino" refers to an -NHR radical where R is alkyl as defined above, e.g. methylamino, ethylamino, n-propylamino, *iso*-propylamino, and the like. Preferably an alkylamino group is a $C_{1-6}$ alkylamino group, and more preferably a $C_{1-4}$ alkylamino group.

As defined herein, the term "aryl" refers to a monovalent monocyclic or bicyclic aromatic hydrocarbon radical of 6 to 10 ring atoms, e.g. phenyl or naphthyl, and the like.

As defined herein, the term "aralkyl" refers to an -(alkylene)-R radical where R is aryl as defined above.

As defined herein, the term "carbamate" refers to a -C(O)NR$^x$R$^y$ radical where R$^x$ and R$^y$ are independently hydrogen, alkyl, haloalkyl, cycloalkyl, cycloalkylalkyl, aryl, aralkyl, heteroaryl, heteroaralkyl, heterocyclyl, or heterocyclylalkyl, each as defined herein, or poly(ethylene glycol), and wherein R$^x$ and R$^y$ are optionally further substituted with one, two, three, four or more substituents independently selected from alkyl, alkoxy, halo, haloalkoxy, -OH, $-NH_2$, alkylamino, -COOH, or alkoxycarbonyl.

As defined herein, the term "cycloalkyl" refers to a cyclic saturated monovalent hydrocarbon radical of three to ten carbon atoms wherein one or two carbon atoms may be replaced by an oxo group, e.g. cyclopropyl, cyclobutyl, cyclopentyl, or cyclohexyl, and the like. Preferably a cycloalkyl group is a $C_{3-10}$ cycloalkyl group, and more preferably a $C_{4-6}$ cycloalkyl group.

As defined herein, the term "cycloalkylalkyl" refers to an -(alkylene)-R radical where R is cycloalkyl as defined above, e.g. cyclopropylmethyl, cyclobutylmethyl, cyclopentylethyl, or cyclohexylmethyl, and the like. As defined herein, the term "halo" refers to fluoro, chloro, bromo, or iodo, preferably fluoro or chloro.

As defined herein, the term "haloalkyl" refers to an alkyl radical as defined above, which is substituted with one or more halogen atoms, preferably one to five halogen atoms, preferably fluorine or chlorine, including those substituted with different halogens, e.g. $-CH_2Cl$, $-CF_3$, $-CHF_2$, $-CH_2CF_3$, $-CF_2CF_3$, $-CF(CH_3)_2$, and the like. Preferably a haloalkyl group is a $C_{1-6}$ haloalkyl group, and more preferably a $C_{1-4}$ haloalkyl group.

As defined herein, the term "haloalkoxy" refers to an -OR radical where R is haloalkyl as defined above, e.g. $-OCF_3$, $-OCHF_2$, and the like.

As defined herein, the term "heteroaryl" refers to a monovalent monocyclic or bicyclic aromatic radical of 5 to 10 ring atoms where one or more, preferably one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like.

As defined herein, the term "heteroaralkyl" refers to an -(alkylene)-R radical where R is heteroaryl as defined above.

As defined herein, the term "heterocycyl" refers to a saturated or unsaturated monovalent monocyclic group of 4 to 8 ring atoms in which one or two ring atoms are heteroatoms selected from N, O, or $S(O)_n$, where n is an integer from 0 to 2, the remaining ring atoms being C. The heterocyclyl ring is optionally fused to a (one) aryl or heteroaryl ring as defined herein provided the aryl and heteroaryl rings are monocyclic. Additionally, one or two ring carbon atoms in the heterocyclyl ring can optionally be replaced by a -CO-group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds, provided that the ring is not aromatic.

As defined herein, the term "heterocycloalkyl" refers to an -(alkylene)-R radical where R is heterocyclyl ring as defined above, e.g. tetrahydrofuranylmethyl, piperazinylmethyl, morpholinylethyl, and the like.

As defined herein, "room temperature" refers to a temperature of 25 °C.

As defined herein, the term "oral cavity" is understood to mean the cavity of the mouth, and includes the inner upper and lower lips, all parts of the inner cheek, the sublingual area under the tongue, the tongue itself, as well as the upper and lower gums and the hard and soft palate.

As defined herein, the term "oral mucosa" is understood to mean the mucous membrane lining the inside of the mouth, and includes (but does not exclusively refer to) mucosa in the buccal, labial, sublingual, ginigival or lip areas, the soft palate and the hard palate.

As defined herein, the term "ambient conditions" is understood to mean a temperature of 25 °C, a pressure of 1 atm and in the presence of air of normal composition (i.e. 78% nitrogen, 21% oxygen, 0.93% argon and 0.04% carbon dioxide).

*Films of the present invention*

The present invention provides films suitable for administration to an oral cavity comprising:

(i) an alginate salt of a monovalent cation or a mixture of alginate salts containing at least one alginate salt of a monovalent cation; and

(ii) an active pharmaceutical ingredient (API) which is a compound of Formula (I) or a pharmaceutically acceptable salt thereof

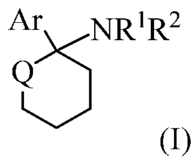

(I)

wherein:

Ar is selected from

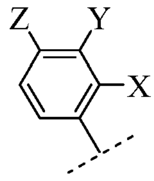

and

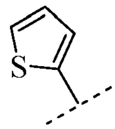

;

X is selected from hydrogen, halo, OH, $NH_2$, methyl, trifluoromethyl and methoxy;
Y is selected from hydrogen, halo, OH, $NH_2$, methyl, trifluoromethyl and methoxy;
Z is selected from hydrogen, halo, OH, $NH_2$, methyl, trifluoromethyl and methoxy;
Q is selected from $-CH_2-$, -CH(OH)-, -CH(Me)-, -CH(OMe)-, -(C=O)-, -(C=S)- and -(C=NR)-, wherein R is selected from hydrogen or $C_{1-6}$ alkyl;
$R^1$ is selected from hydrogen and $C_{1-6}$ alkyl and $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl, or $R^1$ and $R^2$ are linked so as to form a bivalent alkylene moiety having from 3 to 7 carbon atoms.

Preferably, the compound of Formula (I) is ketamine. More preferably, the compound of Formula (I) is esketamine. Alternatively, the compound of Formula (I) is a racemic mixture of arketamine and esketamine. Alternatively, the compound of Formula (I) is arketamine. Alternatively, the compound of Formula (I) is a non-racemic mixture of arketamine and esketamine, preferably wherein the ratio of arketamine:esketmaine is from 1: 100 to 100:1.

The function of said alginate salt of a monovalent cation or mixture of alginate salts containing at least one alginate salt of a monovalent cation within the film is to act as a film-forming agent. As used herein, the term "film-forming agent" refers to a chemical or group of chemicals that form a pliable, cohesive and continuous covering when applied to a surface.

Alginate, the salt of alginic acid, is a linear polysaccharide naturally produced by brown seaweeds (*Phaeophyceae,* mainly *Laminaria*). Typically the alginate employed in the present invention comprises from 100 to 3000 monomer residues linked together in a flexible chain. These residues are of two types, namely $\beta$-(1,4)-linked D-mannuronic acid (M) residues and $\alpha$-(1,4)-linked L-guluronic acid (G) residues. Typically, at physiological pH, the carboxylic acid group of each residue in the polymer is ionised. The two residue types are epimers of one another, differing only in their stereochemistry at the C5 position, with D-mannuronic acid residues being enzymatically converted to L-guluronic acid residues after polymerization. However, in the polymer chain the two residue types give rise to very different conformations: any two adjacent D-mannuronic acid residues are $^4C_1$-diequatorially linked whilst any two adjacent L-guluronic acid residues are $^4C_1$-diaxially linked, as illustrated in Formula (II) below.

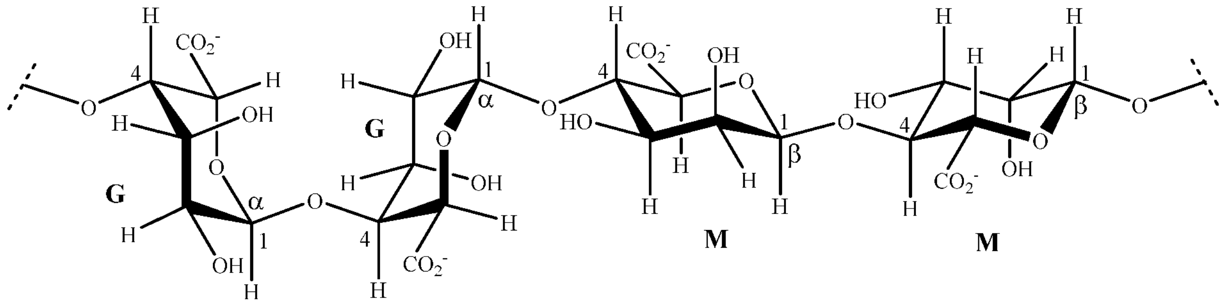

(II)

Typically in the alginate polymer, the residues are organised in blocks of identical or strictly alternating residues, e.g. MMMMM..., GGGGG... or GMGMGM.... Different monovalent and polyvalent cations may be present as counter ions to the negatively-charged carboxylate groups of the D-mannuronic acid and L-guluronic acid residues of the alginate polymer. Typically, the film comprises an alginate salt wherein the counter ions of the alginate polymer are monovalent cations. The cations which are the counterions of a single alginate polymer molecule may all be the same as one another or may be different to one another. Preferably, the counterions of the alginate polymer are selected from $Na^+$, $K^+$ and $NH_4^+$. More preferably, the counterions of the alginate polymer are $Na^+$. Alternatively, the film may comprise a mixture of alginate salts containing at least one alginate salt of a monovalent cation. The mixture of alginate salts may comprise an alginate salt of a cation selected from $Na^+$, $K^+$ and $NH_4^+$. Thus, typically, the alginate chains are not cross-linked, i.e. there is no, or substantially no, ionic cross-linking between the alginate strands. Ionic cross-linking of alginates results from the presence of divalent counterions. "Substantially no" cross-linking can be taken to mean that fewer than 10% by weight of the alginate polymer chains in the film are cross-linked, preferably fewer than 5% by weight, more preferably fewer than 2% by weight, still more preferably fewer than 1% by weight, yet more preferably fewer than 0.5% by weight, and most preferably fewer than 0.1% by weight. Thus, preferably, the films of the present invention comprise no alginate salts of a divalent cation.

Alignates are commercially available and the skilled person is able to synthesise them using routine techniques.

Typically, the film comprises an alginate composition which has a dynamic viscosity, as measured on a 10% aqueous solution (w/w) thereof at a temperature of 20 °C with a Brookfield LVF viscometer (obtained from Brookfield Engineering Laboratories, Inc.), using a spindle No. 2 at a shear rate of 20 rpm, of 100-1000 mPa.s, or 200-800 mPa.s, or 300-700 mPa.s.

The film comprises an alginate composition having a mean guluronate (G) content of from 65 to 75% by weight. The film comprises an alginate composition having a mean mannuronate (M) content of from 25 to 35% by weight. The film comprises an alginate composition having a weight average molecular weight ranging from 30,000 g/mol to 90,000 g/mol, such as from 35,000 g/mol to 85,000 g/mol, or from 40,000 g/mol to 70,000 g/mol, or from 40,000 g/mol to 50,000 g/mol. The film comprises an alginate composition having a mean guluronate (G) content of from 65 to 75%, a mean mannuronate (M) content of from 25 to 35%, and a weight average molecular weight ranging from 30,000 g/mol to 90,000 g/mol. Without wishing to be bound by any particular theory, it is believed that it is a combination of both (a) the particular mean relative proportions of mannuronate and guluronate in the alginate composition and (b) the particular weight average molecular weight of the alginate composition that endow the film with its desirable bioadhesive properties.

The alginate salt of a monovalent cation or the mixture of alginate salts containing at least one alginate salt of a monovalent cation may be the sole film-forming agent present in the film. Alternatively, the film may comprise one or more further film-forming agents in addition to the alginate salt of a monovalent cation or the mixture of alginate salts containing at least one alginate salt of a monovalent cation.

It is preferred that the film comprises Protanal® LFR 5/60 or Protanal® LF 10/60 (both commercially available sodium alginate products from FMC BioPolymer) as the alginate salt. Protonal® LFR 5/60 is a low molecular weight and low viscosity sodium alginate extracted from the stem of *Laminaria hyperborean.* Protanal® LF 10/60 is a sodium alginate having a G/M % ratio of 65-75/25-35 and a viscosity of from 20-70 mPas as measured on a 1% aqueous solution thereof at a temperature of 20 °C with a Brookfield LVF viscometer, using a spindle No. 2 at a shear rate of 20 rpm. Protanal® LF 10/60 has both a higher weight average molecular weight and a higher viscosity than Protanal® LFR 5/60.

Without wishing to be bound by any particular theory, a film comprising a higher viscosity alginate salt is believed to have a longer residence time (i.e. dissolving time) after application to the oral cavity *via* adhesion to a mucous membrane of said cavity than a film comprising a lower viscosity alginate salt of a similar thickness. It is contemplated that the viscosity of the alginate composition within the film may be adjusted by mixing any number of alginates having different viscosities. Typically, a film of about 1 mm thickness comprising Protanal® LFR 5/60 as the sole alginate component has a residence time of approximately 3-10 minutes after adhesion to a mucous membrane of the oral cavity.

In contrast, a film of about 1 mm thickness comprising Protanal® LF 10/60 as the sole alginate component has a residence time of approximately 30 minutes after adhesion to a mucous membrane of the oral cavity.

Therefore, if a long residence time of the film within the oral cavity is desired, it is generally preferred that the film comprises Protanal® LF 10/60 as the alginate salt. However, compared to films comprising Protanal® LFR 5/60 as the alginate salt, films comprising Protanal® LF 10/60 as the alginate salt typically exhibit inferior adhesion properties when applied to a mucous membrane of the oral cavity. More generally, it is believed that film-forming agents having longer average chain lengths exhibit poorer adhesion to mucosa than film-forming agents having shorter average chain lengths. Without wishing to be bound by any particular theory, it is believed that better mucoadhesion of a film to the mucous membrane of the oral cavity enables a more efficient delivery of any active ingredients contained within the film to their site of action. Therefore, if a long residence time of the film within the oral cavity is not particularly necessary, it may be preferable to use Protanal® LFR 5/60 as the alginate salt.

It is particularly preferred that the film comprises Protanal® LFR 5/60 as the alginate salt.

The film may also comprise a film-forming agent other than the alginate salt of a monovalent cation or the mixture of alginate salts containing at least one alginate salt of a monovalent cation. Such other film-forming agents include agents such as poly(vinyl pyrrolidone) (PVP), hydroxypropylmethylcellulose (HPMC), poloxamers, pullulan, and so forth. However, if any other film-forming agent is present in the film in addition to the alginate salt of a monovalent cation or the mixture of alginate salts containing at least one alginate salt of a monovalent cation, then typically the alginate salt of a monovalent cation or the mixture of alginate salts containing at least one alginate salt of a monovalent cation will be present in the film in excess over any other film-forming agent present. Preferably, the ratio (by weight) of the alginate salt of a monovalent cation or the mixture of alginate salts containing at least one alginate salt of a monovalent cation present in the film to the combined total of all other film-forming agents (such as PVP, HPMC, poloxamers and/or pullulan) present in the film is 1:1 or greater, or 2:1 or greater, or 3:1 or greater, or 4:1 or greater, or 5:1 or greater, or 10:1 or greater, or 20:1 or greater, or 50:1 or greater, or 100:1 or greater, or 200:1 or greater. Preferably, the alginate salt of a monovalent cation or the mixture of alginate salts containing at least one alginate salt of a monovalent cation will constitute at least 50% by weight of the total of the film-forming agents present in the film, more preferably at least 60% by weight, at least 70% by weight, at least 80% by weight, at least 90% by weight, at least 95% by weight, at least 98% by weight, at least 99% by weight, or at least 99.5% by weight of the total of the film-forming agents present in the film.

Preferably, the alginate salt of a monovalent cation or the mixture of alginate salts containing at least one alginate salt of a monovalent cation is substantially the only film-forming agent present in the film. In some cases, the alginate salt of a monovalent cation or the mixture of alginate salts containing at least one alginate salt of a monovalent cation is the only film-forming agent present in the film. Alternatively, the film does not comprise any, or substantially any, poly(vinyl pyrrolidone). Alternatively, the film does not comprise any, or substantially any, pullulan. Alternatively, the film does not comprise any, or substantially any, hydroxypropylmethylcellulose. Alternatively, the film does not comprise any, or substantially any, poloxamers.

As used herein, a reference to a film that does not comprise "substantially any" of a specified component refers to a film that may contain trace amounts of the specified component, provided that the specified component does not materially affect the essential characteristics of the film. Typically, therefore, a film that does not comprise substantially any of a specified component contains less than 5 wt% of the specified component, preferably less than 1 wt% of the specified component, most preferably less than 0.1 wt% of the specified component.

It is a finding of the present invention that the use of an alginate salt of a monovalent cation or a mixture of alginate salts containing at least one alginate salt of a monovalent cation as the film-forming agent has benefits over the use of alternative film-forming agents, such as PVP, HPMC, poloxamers and/or pullulan. In particular, the use of alginate as the primary film-forming agent ensures that the films of the present invention have superior adhesive properties over films comprising primarily other film-forming agents such as PVP, HPMC, poloxamers or pullulan. The films of the present invention are bioadhesive; that is to say that the films of the present invention can firmly adhere to a moist surface (i.e. mucosa) in the oral cavity of a mammal subject before it has fully dissolved. Films in which alginate is not the primary film-forming agent do not generally have this desirable property. A further advantageous finding of the present invention is that the choice of alginate as the primary film-forming agent enables therapeutically effective doses of an active pharmaceutical ingredient (e.g., ketamine) to be loaded into the films whilst retaining homogeneity and other desirable physical properties of the films.

Without wishing to be bound by any particular theory, it is believed that one of the reasons that alginate is a preferable film-forming agent to, e.g., PVP, HPMC, poloxamers and pullulan, is that the negatively charged alginate salt may act as a counterion to a positively charged amine salt of the compound of Formula (I) (i.e. the API), thus producing a solid, amorphous dispersion during the film manufacture (i.e. enabling the production of clear film with desirable physical characteristics).

Typically, the film comprises from 15% to 99% by weight of the alginate salt of a monovalent cation or the mixture of alginate salts containing at least one alginate salt of a monovalent cation, preferably from 18% to 95% by weight, more preferably from 20% to 93% by weight, still more preferably from 25% to 91% by weight, and most preferably

from 30% to 90% by weight.

The film according to the present invention may also contain a residual water content. Typically, the film comprises from 0% to 20% by weight of residual water. More typically, the film comprises from 5% to 15% by weight of residual water. Preferably, the film comprises from 9% to 11% by weight of residual water. Most preferably, the film comprises about 10% by weight of residual water. Typically, the low water content of the film distinguishes the film from pastes or gels (e.g. hydrogels), which typically have higher water contents. Thus, typically, the film of the present invention is not a paste. Typically, the film of the present invention is not a gel.

The film according to the present invention also comprises an active pharmaceutical ingredient (API) which is a compound of Formula (I) or a pharmaceutically acceptable salt thereof

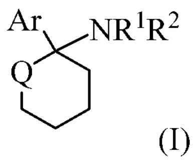

(I)

wherein:

Ar is selected from

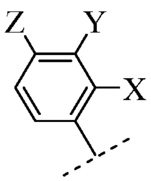

and

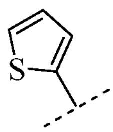

;

X is selected from hydrogen, halo, OH, $NH_2$, methyl, trifluoromethyl and methoxy;
Y is selected from hydrogen, halo, OH, $NH_2$, methyl, trifluoromethyl and methoxy;
Z is selected from hydrogen, halo, OH, $NH_2$, methyl, trifluoromethyl and methoxy;
Q is selected from -$CH_2$-, -CH(OH)-, -CH(Me)-, -CH(OMe)-, -(C=O)-, -(C=S)- and -(C=NR)-, wherein R is selected from hydrogen or $C_{1-6}$ alkyl;
$R^1$ is selected from hydrogen and $C_{1-6}$ alkyl and $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl, or $R^1$ and $R^2$ are linked so as to form a bivalent alkylene moiety having from 3 to 7 carbon atoms.

Thus, typically $R^1$ is selected from hydrogen and $C_{1-6}$ alkyl. Typically, $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl. Alternatively, $R^1$ and $R^2$ are linked so as to form a bivalent alkylene moiety having from 3 to 7 carbon atoms.

Typically, Ar is

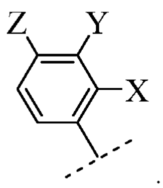

.

In such aspects, typically X is selected from hydrogen, halo, OH, $NH_2$, methyl and methoxy. Preferably, X is selected from hydrogen, halo, OH, methyl and methoxy. More preferably, X is selected from hydrogen, halo, OH and methoxy. Yet more preferably, X is selected from hydrogen, halo and methoxy. Still more preferably, X is selected from hydrogen and halo. Most preferably, X is halo, and preferably is chloro.

In such aspects, typically Y is selected from hydrogen, halo, OH, $NH_2$, methyl and methoxy. Preferably, Y is selected from hydrogen, halo, OH, methyl and methoxy. More preferably, Y is selected from hydrogen, halo, OH and methoxy. Yet more preferably, Y is selected from hydrogen, halo and methoxy. Still more preferably, Y is selected from hydrogen and methoxy. Most preferably, Y is hydrogen.

In such aspects, typically Z is selected from hydrogen, halo, OH, $NH_2$, methyl and methoxy. Preferably, Z is selected from hydrogen, halo, OH, methyl and methoxy. More preferably, Z is selected from hydrogen, halo, OH and methoxy. Yet more preferably, Z is selected from hydrogen, halo and methoxy. Still more preferably, Z is selected from hydrogen and methoxy. Most preferably, Z is hydrogen.

In such aspects, typically at least one of X, Y and Z are hydrogen, e.g. two or three of X, Y and Z are hydrogen. Preferably, two of X, Y and Z are hydrogen. For example, in some embodiments X and Z are hydrogen, and Y is methoxy. Preferably, Y and Z are hydrogen, and X is halo, preferably chloro.

In such aspects, typically $R^1$ is selected from hydrogen, methyl or ethyl. Preferably, $R^1$ is hydrogen. Typically, $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl. Preferably, $R^2$ is selected from hydrogen, unsubstituted $C_{1-6}$ alkyl or $C_{1-6}$ alkenyl. More preferably, $R^2$ is selected from hydrogen and $C_{1-6}$ alkyl. Yet more preferably, $R^2$ is selected from hydrogen, methyl and ethyl. Most preferably, $R^2$ is methyl.

In such aspects, preferably at least one of $R^1$ and $R^2$ is hydrogen. More preferably, $R^1$ is hydrogen and $R^2$ is selected from $C_{1-6}$ alkyl optionally substituted with halo, hydroxyl, $C_{1-4}$ alkoxy, amino or $C_{1-4}$ alkylamino, and $C_{1-6}$ alkenyl. Yet more preferably, $R^1$ is hydrogen and $R^2$ is selected from hydrogen, unsubstituted $C_{1-6}$ alkyl or $C_{1-6}$ alkenyl. Still more preferably, $R^1$ is hydrogen and $R^2$ is selected from hydrogen and $C_{1-6}$ alkyl. Even more preferably, $R^1$ is hydrogen and $R^2$ is selected from hydrogen, methyl and ethyl. Most preferably, $R^1$ is hydrogen and $R^2$ is methyl.

In such aspects, $R^1$ and $R^2$ may alternatively be linked so as to form a bivalent alkylene moiety having from 3 to 7 carbon atoms. In this case, preferably $R^1$ and $R^2$ are linked so as to form a bivalent alkylene moiety having from 4 to 6 carbon atoms, most preferably 5 carbon atoms. Typically, in these embodiments, Q is $-CH_2-$.

In such aspects, typically Q is selected from $-CH_2-$, -(C=O)-, -(C=S)- and -(C=NR)-, wherein R is selected from hydrogen or $C_{1-6}$ alkyl. Preferably, Q is selected from -(C=O)-, -(C=S)- and -(C=NR)-, and most preferably Q is -(C=O)-.

Alternatively, Ar is

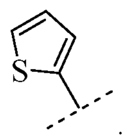

In these aspects, typically $R^1$ is selected from hydrogen, methyl or ethyl. Preferably, $R^1$ is hydrogen. Typically, $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl. Preferably, $R^2$ is selected from hydrogen, unsubstituted $C_{1-6}$ alkyl or $C_{1-6}$ alkenyl. More preferably, $R^2$ is selected from hydrogen and $C_{1-6}$ alkyl. Yet more preferably, $R^2$ is selected from hydrogen, methyl and ethyl. Most preferably, $R^2$ is ethyl.

In these aspects, preferably at least one of $R^1$ and $R^2$ is hydrogen. More preferably, $R^1$ is hydrogen and $R^2$ is selected from $C_{1-6}$ alkyl optionally substituted with halo, hydroxyl, $C_{1-4}$ alkoxy, amino or $C_{1-4}$ alkylamino, and $C_{1-6}$ alkenyl. Yet more preferably, $R^1$ is hydrogen and $R^2$ is selected from hydrogen, unsubstituted $C_{1-6}$ alkyl or $C_{1-6}$ alkenyl. Still more preferably, $R^1$ is hydrogen and $R^2$ is selected from hydrogen and $C_{1-6}$ alkyl. Even more preferably, $R^1$ is hydrogen and $R^2$ is selected from hydrogen, methyl and ethyl. Most preferably, $R^1$ is hydrogen and $R^2$ is methyl.

In these aspects, $R^1$ and $R^2$ may alternatively be linked so as to form a bivalent alkylene moiety having from 3 to 7 carbon atoms. In this case, preferably $R^1$ and $R^2$ are linked so as to form a bivalent alkylene moiety having from 4 to 6 carbon atoms, most preferably 5 carbon atoms. Typically, in these embodiments, Q is $-CH_2-$.

In these aspects, typically Q is selected from $-CH_2-$, -(C=O)-, -(C=S)- and -(C=NR)-, wherein R is selected from hydrogen or $C_{1-6}$ alkyl. Preferably, Q is selected from -(C=O)-, -(C=S)- and -(C=NR)-, and most preferably Q is -(C=O)-.

Preferably, the compound of Formula (I) is selected from ketamine, tiletamine or a pharmaceutically acceptable salt thereof.

More preferably, the compound of Formula (I) is ketamine or a pharmaceutically acceptable salt thereof. The structure of ketamine is provided below as Formula (III).

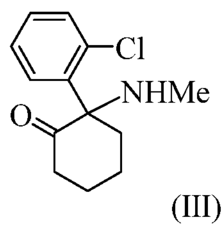

(III)

The compounds of Formula (I) may contain one or more stereogenic centres. For example, when substituent Q in Formula (I) is other than $-CH_2-$, the carbon atom to which the Q, aryl and $NR^1R^2$ groups are bonded is a stereogenic centre. Similarly, when substituent Q is -CH(OH)- or -CH(Me)-, the carbon atom in Q that forms part of the cyclohexane-derived ring is a stereogenic centre. Certain compounds of Formula (I) may therefore be isolated in optically active or racemic forms. It is well-known in the art how to prepare optically active forms, such as by resolution of materials. For the avoidance of doubt, Formula (I) encompasses all enantiomeric, diastereomeric, and racemic forms of the compounds thereof, as well as all mixtures of enantiomers and diastereomers of the compounds thereof.

Thus, for example, when the compound of Formula (I) is ketamine, this means that the compound of Formula (I) may be (S)-ketamine (commonly referred to as esketamine), (R)-ketamine (commonly referred to as arketamine), or a mixture of esketamine and arketamine.

Most preferably, the compound of Formula (I) is esketamine or a racemic mixture of esketamine and arketamine, or a pharmaceutically acceptable salt thereof. Thus, in a preferable embodiment, the compound of Formula (I) is esketamine or a pharmaceutically acceptable salt thereof. In an alternative preferable embodiment, the compound of Formula (I) is a racemic mixture of esketamine and arketamine. The structure of esketamine is provided below as Formula (IV).

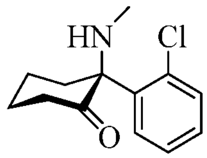

(IV)

Alternatively, the compound of Formula (I) may be arketamine. The structure of arketamine is provided below as Formula (IVa).

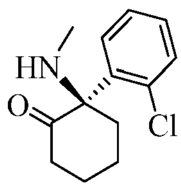

(IVa)

The API may be a pharmaceutically acceptable polymorph, co-crystal, hydrate or solvate of the compound of Formula (I) or pharmaceutically acceptable salt thereof, preferably a pharmaceutically acceptable polymorph, co-crystal, hydrate or solvate of ketamine or a pharmaceutically acceptable salt thereof, e.g. a pharmaceutically acceptable polymorph, co-crystal, hydrate or solvate of arketamine, esketamine, or a mixture of arketamine and esketamine, or a pharmaceutically acceptable salt thereof, more preferably a pharmaceutically acceptable polymorph, co-crystal, hydrate or solvate of esketamine or a pharmaceutically acceptable salt thereof.

Alternatively, the API may be a prodrug of a compound of Formula (I) or a pharmaceutically acceptable salt thereof, preferably a prodrug of ketamine, e.g. a prodrug of arketamine, esketamine or a mixture of arketamine and esketamine, more preferably a prodrug of esketamine. The term "prodrug" of a compound of Formula (I), as used herein, refers to any compound or pharmaceutically acceptable salt thereof which, after administration to the human body, may be metabolised *in vivo* to a compound of Formula (I). Preferred prodrugs of a compound of Formula (I) include N-acyl, N-alkoxycarbonyl and N-carbamate derivatives of a compound of Formula (I), i.e. compounds of Formula (I) in which one of $R^1$ or $R^2$ is acyl, alkoxycarbonyl or carbamate. Particularly preferred prodrugs are prodrugs of ketamine, more preferably a compound of Formula (V):

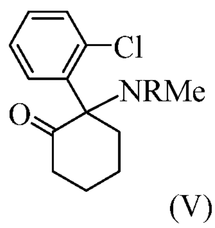

(V)

wherein R is acyl, alkoxycarbonyl or carbamate.

In a first embodiment of the present invention, the API is a neutral compound of Formula (I), preferably the free base form of ketamine. In this embodiment, the film typically also contains an acid $H_xA$, wherein A is a counterion having an ionic radius of 2.65 Å or greater, and x is a positive integer which is equal to the charge on the counterion A. It has been surprisingly found that the presence of such an acid in the films of the present invention prevents the growth of ketamine crystals in the film, thus increasing the film stability during storage under ambient conditions. Without wishing to be bound by any particular theory, it is thought that the electronegativity and/or size of the counterion A may play a role in this effect.

Preferably, the counterion A has an ionic radius of 2.70 Å or greater, more preferably 2.75 Å or greater, and most preferably 2.80 Å or greater, e.g. 3.0 Å or greater, or 3.5 Å or greater.

Typically, the counterion A has a van der Waals volume (molecular volume) of 45 $Å^3$ or greater, preferably 50 $Å^3$ or greater, and most preferably 55 $Å^3$ or greater, e.g. 60 $Å^3$ or greater, 75 $Å^3$ or greater or 100 $Å^3$ or greater.

Preferably, the acid $H_xA$ is a weak acid having a $pK_a$ greater than 0.

Preferably, the acid $H_xA$ is selected from acetic acid, ascorbic acid, phosphoric acid, citric acid, tartaric acid, acrylic acid, poly(acrylic) acid, iodic acid, malic acid, methanesulfonic acid and combinations thereof. Most preferably, the acid $H_xA$ is phosphoric acid.

Typically, the amount of acid added to the film is an amount necessary to achieve a pH of 3.0 or higher, preferably 3.5 or higher, most preferably 4.0 or higher, e.g. about 4.0, when the acid is added to a solution of water containing the desired amount of compound of Formula (I) as API (e.g. ketamine).

In some aspects of this embodiment, the film further comprises an additive selected from xylitol, a cyclodextrin, poly(vinyl pyrrolidone), hydroxypropylmethylcellulose, poly(acrylic acid) and pullulan. These additives have also surprisingly been found to suppress ketamine crystal growth in alginate films. A particularly preferred additive in this regard is poly(acrylic acid). In these aspects, the ratio of API: additive is typically 1:1 or greater, for instance from 1:1 to 1:1000, typically from 1:1 to 1:500, preferably from 1:1 to 1:200, more preferably from 1:1 to 1:100, still more preferably from 1:1 to 1:50, yet more preferably from 1:1 to 1:20, even more preferably from 1:1 to 1:10, and most preferably from 1:1 to 1:5, e.g. from 1:1 to 1:4, from 1:1 to 1:3 or from 1:1 to 1:2. Alternatively, the ratio of API:additive may be less than 1:1, for example from 0.1:1 to 1:1, from 0.2:1 to 1:1, or from 0.5:1 to 1:1. In these aspects, the ratio of alginate: additive is typically from 1:100 to 100:1, preferably from 1:50 to 50:1, more preferably from 1:10 to 10:1, yet more preferably from 1:5 to 5:1, still more preferably from 1:2 to 2:1, and most preferably from 1:1 to 2:1.

In this embodiment, the API may be present within the film in varying amounts. Typically, the film comprises from 0.001% to 75% by weight of the API, preferably from 0.01% to 60% by weight of the API, more preferably from 0.15% to 50% by weight of the API, still more preferably from 0.2% to 45% by weight of the API and most preferably from 0.25% to 40% by weight of the API.

In a second embodiment of the present invention, the API is a pharmaceutically acceptable salt of the compound of Formula (I), preferably a pharmaceutically acceptable salt of ketamine. As the compounds of Formula (I) contain a basic nitrogen atom, typically the pharmaceutically acceptable salt of the compound of Formula (I) is selected from acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, phosphate, aluminium, ammonium, calcium, copper, ferric, ferrous, lithium, magnesium, manganic, manganous, potassium, sodium, zinc, arginine, betaine, caffeine, choline, *N,N'*- dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine and tromethamine salts of the compound of Formula (I). Preferred salt forms of the compound of Formula (I) include acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, and phosphate salts of the compound of Formula (I). More preferred salt forms of the compound of Formula (I) include hydrochloride and hydrobromide salts of the compound of Formula (I), in particular hydrochloride salts of the compound of Formula (I).

As defined herein, the term "compound of Formula (I)" refers to the form of the compound of Formula (I) in which the molecules are present in neutral (i.e. unionized) form. The term "pharmaceutically acceptable salt of the compound of Formula (I)" refers to any salt of the compound of Formula (I). For example, when the compound of Formula (I) is ketamine, the term "pharmaceutically acceptable salt of ketamine" refers to any salt of ketamine in which the amine group is protonated.

Typically, in this embodiment, the API is a pharmaceutically acceptable salt of ketamine selected from acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, and phosphate salts of ketamine. Preferred salt forms of ketamine include hydrochloric acid salts and hydrobromide salts of ketamine, and most preferably the pharmaceutically acceptable salt of ketamine is a hydrochloride salt of ketamine.

Typically, in this embodiment, the API is a pharmaceutically acceptable salt of esketamine selected from acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, and phosphate salts of esketamine. Preferred salt forms of esketamine include hydrochloric acid salts and hydrobromide salts of esketamine, and most preferably the pharmaceutically acceptable salt of esketamine is a hydrochloride salt of esketamine.

Alternatively, in this embodiment, the API is a pharmaceutically acceptable salt of a racemic mixture of arketamine and esketamine selected from acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, and phosphate salts of a racemic mixture of arketamine and esketamine. Preferred salt forms of the racemic mixture of arketamine and esketamine include hydrochloric acid salts and hydrobromide salts of the racemic mixture of arketamine and esketamine, and most preferably the pharmaceutically acceptable salt of the racemic mixture of arketamine and esketamine is a hydrochloride salt of the racemic mixture of arketamine and esketamine.

Altermatively, in this embodiment, the API is a pharmaceutically acceptable salt of arketamine selected from acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, and phosphate salts of arketamine. Preferred salt forms of arketamine include hydrochloric acid salts and hydrobromide salts of arketamine, and most preferably the pharmaceutically acceptable salt of arketamine is a hydrochloride salt of arketamine.

In this embodiment, the film typically also contains an additive selected from xylitol, a cyclodextrin, poly(vinyl pyrrolidone), hydroxypropylmethylcellulose, poly(acrylic acid) and pullulan. These additives have also surprisingly been found to suppress ketamine crystal growth in alginate films. A particularly preferred additive in this regard is poly(acrylic acid).

The ratio of API:additive in such films may vary. Typically, though, the ratio of API:additive is 1:1 or greater, for instance from 1:1 to 1:1000, typically from 1:1 to 1:500, preferably from 1:1 to 1:200, more preferably from 1:1 to 1:100, still more preferably from 1:1 to 1:50, yet more preferably from 1:1 to 1:20, even more preferably from 1:1 to 1:10, and most preferably from 1:1 to 1:5, e.g. from 1:1 to 1:4, from 1:1 to 1:3 or from 1:1 to 1:2. Alternatively, the ratio of API:additive may be less than 1:1, for example from 0.1:1 to 1:1, from 0.2:1 to 1:1, or from 0.5:1 to 1:1.

The ratio of alginate: additive in such films may also vary. Typically, though, the ratio of alginate: additive is from 1:10 to 50:1, preferably from 1:10 to 10:1, more preferably from 1:5 to 5:1, still more preferably from 1:2 to 2:1, and most preferably from 1:1 to 2:1.

In this embodiment, the API may be present within the film in varying amounts. Typically, the film comprises from 0.001% to 75% by weight of the API, preferably from 0.01% to 60% by weight of the API, more preferably from 0.15% to 50% by weight of the API, still more preferably from 0.2% to 45% by weight of the API and most preferably from 0.25% to 40% by weight of the API.

In some aspects of this embodiment, the film further contains an acid $H_xA$, wherein A is a counterion having an ionic radius of 2.65 Å or greater, preferably 2.70 Å or greater, more preferably 2.75 Å or greater, and most preferably 2.80 Å or greater, e.g. 3.0 Å or greater, or 3.5 Å or greater. Optionally, the counterion A has a van der Waals volume (molecular volume) of 45 $Å^3$ or greater, preferably 50 $Å^3$ or greater, and most preferably 55 $Å^3$ or greater, e.g. 60 $Å^3$ or greater, 75 $Å^3$ or greater or 100 $Å^3$ or greater. Preferably, the acid $H_xA$ is selected from acetic acid, ascorbic acid, phosphoric acid, citric acid, tartaric acid, acrylic acid, poly(acrylic) acid, iodic acid, malic acid, methanesulfonic acid and combinations thereof. Most preferably, the acid $H_xA$ is phosphoric acid. Typically, the amount of acid added to the film is an amount necessary to achieve a pH of 3.0 or higher, preferably 3.5 or higher, most preferably 4.0 or higher, e.g. about 4.0, when the acid is added to a solution of water containing the desired amount of compound of Formula (I) as API (e.g. ketamine).

Typically (in either embodiment of the invention), the compound of Formula (I) or pharmaceutically acceptable salt thereof is the only API present in the film. However, the film may alternatively comprise one or more further active pharmaceutical ingredients in addition to the compound of Formula (I) or pharmaceutically acceptable salt thereof.

Preferably, a film of the present invention (of either embodiment) comprises from 15% to 99% by weight of the alginate salt of a monovalent cation or the mixture of alginate salts containing at least one alginate salt of a monovalent cation, from 0% to 20% by weight of water, and from 0.001% to 75% by weight of the API. More preferably, the film comprises from 20% to 93% by weight of the alginate salt of a monovalent cation or the mixture of alginate salts containing at least one alginate salt of a monovalent cation, from 5% to 15% by weight of water, and from 0.15% to 50% by weight of the API. Even more preferably, the film comprises from 25% to 91% by weight of the alginate salt of a monovalent cation or the mixture of alginate salts containing at least one alginate salt of a monovalent cation, from 9% to 11% by weight of water, and from 0.2% to 45% by weight of the API.

A film according to the present invention (of either embodiment) may optionally further comprise other components in addition to those discussed above. Typically, a film according to the present invention further comprises one or more of the following:

(i) at least one pharmaceutically acceptable solvent;
(ii) at least one buffering component;
(iii) at least one excipient, such as one or more plasticizers, fillers, taste-masking agents or flavouring agents;
(iv) at least one acidifying agent or basifying agent;
(v) at least one permeation enhancer;
(vi) a self-emulsifying drug delivery system (SEDDS), such as a self-microemulsifying drug delivery system (SMEDDS) or a self-nanoemulsifying drug delivery system (SNEDDS);
(vii) at least one chelating agent;
(viii) at least one antioxidant;
(ix) at least one antimicrobial agent; and
(x) at least one inorganic salt.

The film may additionally comprise any pharmaceutically acceptable solvent. Such a solvent may be a non-aqueous solvent, or a combination of water and a non-aqueous solvent. Examples of non-aqueous solvents should be non-toxic and include, but are not limited to, ethanol, acetone, benzyl alcohol, diethylene glycol monoethyl ether, glycerine, hexylene glycol, isopropyl alcohol, polyethylene glycols, methoxypolyethylene glycols, diethyl sebacate, dimethyl isosorbide, propylene carbonate, dimethyl sulfoxide, transcutol, triacetin, fatty acid esters, and oils such as soybean oil, peanut oil, olive oil, palm oil, rapeseed oil, corn oil, coconut oil, other vegetable oils and the like.

The film may additionally comprise any suitable buffering component. A "buffering component", as defined herein, refers to any chemical entity, which when dissolved in solution, enables said solution to resist changes in its pH following the subsequent addition of either an acid or a base. A suitable buffering component for use in the film of the present invention would be a buffering component which is an effective buffer within a pH range of from 3.0 to 5.5. Preferably, said buffering component is an effective buffer within a pH range of from 3.8 to 5.5. Examples of suitable buffering components include, but are not limited to: phosphates, sulfates, citrates and acetates. The buffer may be a salt of a monovalent cation, such as sodium, potassium or ammonium salts. Particularly preferred buffering components include citric acid and sodium dihydrogen phosphate. Without wishing to be bound by any particular theory, it is believed that ketamine has a low stability towards oxidation at a pH of greater than 5.5. Further, without wishing to be bound by any particular theory, it is believed that alginate tends to gel at a pH of less than 3.8.

The film may comprise from 0.1% to 10% by weight of the buffering component, typically 0.2% to 8% by weight, typically from 0.3% to 6% by weight, typically from 0.5% to 5% by weight. Alternatively, the film may not additionally comprise a buffering component.

The film may additionally comprise any suitable excipient, such as one or more fillers or plasticizers. The film may comprise both a plasticizer and a filler. Alternatively, the film may comprise just one of a plasticizer or a filler. It is preferred that the film comprises a plasticizer. Under some circumstances it may be desirable that the film does not comprise a filler. It is particularly preferred that the film comprises a plasticizer but does not comprise a filler. The film may additionally include a taste-masking agent or a flavouring agent. The taste-masking agent may be a sweetener.

The plasticizer, when present, may be selected from polyethylene glycol, glycerol, sorbitol, xylitol, and a combination thereof. Typically, the film comprises a plasticizer which is selected from glycerol, sorbitol, xylitol, and a combination thereof. Preferably, the film comprises a plasticizer which is selected from glycerol, sorbitol, and a combination thereof. More preferably, the film comprises both glycerol and sorbitol as plasticizers. Most preferably, the film comprises glycerol, sorbitol and xylitol. The film may comprise from 0% to 40% by weight of each plasticizer present, preferably from 1% to 35% by weight of each plasticizer, more preferably from 2% to 30% by weight of each plasticizer, and most preferably from 3% to 25% by weight of each plasticizer. Without wishing to be bound by any particular theory, it is believed that the addition of plasticizers, e.g. a combination of glycerol, sorbitol and xylitol, increases the flexibility and pliability of the films, reducing brittleness. It is believed this makes the films easier to handle and use.

The filler, when present, may be e.g. microcrystalline cellulose or titanium dioxide. A suitable amount of filler may be from 0% to 20% by weight, e.g. from 0.1% to 10% by weight, of the total pharmaceutical composition.

The flavouring agent, when present, may for example be selected from acacia, anise oil, caraway oil, cardamom, cherry syrup, cinnamon, citric acid syrup, clove oil, cocoa, coriander oil, ethyl vanillin, fennel oil, ginger, glycerine, glycyrrhiza, honey, lavender oil, lemon oil, mannitol, nutmeg oil, orange oil, orange flower water, peppermint oil, raspberry, rose oil, rosewater, rosemary oil, sarsaparilla syrup, spearmint oil, thyme oil, tolu balsam syrup, vanilla, wild cherry syrup, and mixtures thereof. The film may comprise from 0.001% to 10% by weight of each flavouring agent present, preferably from 0.01% to 5% by weight of each flavouring agent, and most preferably from 0.1% to 3% by weight of each flavouring agent.

The film may additionally comprise an acidifying agent or a basifying agent. An "acidifying agent", as defined herein, refers to a chemical compound that alone or in combination with other compounds can be used to acidify a pharmaceutical composition. A "basifying agent", as defined herein, refers to a chemical compound that alone or in combination with other compounds can be used to basify a pharmaceutical composition.

Typically, the film comprises a basifying agent. Typically, the basifying agent is an alkali. Examples of suitable basifying agents include, but are not limited to: sodium hydroxide, lithium hydroxide, potassium hydroxide, magnesium hydroxide, and calcium hydroxide. A preferable basifying agent is sodium hydroxide. Alternatively, the film may comprise an acidifying agent. Examples of suitable acidifying agents include, but are not limited to: acetic acid, dehydro acetic acid, alginic acid, ascorbic acid, benzoic acid, boric acid, citric acid, edetic acid, hydrochloric acid, isostearic acid, lactic acid, nitric acid, oleic acid, phosphoric acid, malic acid, methanesulfonic acid, sorbic acid, stearic acid, sulfuric acid, tartaric acid, and undecylenic acid. A preferable acidifying agent is phosphoric acid.

A film according to the present invention is produced *via* the drying of a film-forming solution *(vide infra).* Typically, a sufficient amount of acidifying agent or basifying agent is added to adjust the pH of the film-forming solution (before this is dried to form the film) to a pH of from 3.0 to 5.5, preferably to a pH of from 3.8 to 5.5.

The film may additionally comprise any suitable permeation enhancer. A "permeation enhancer", as defined herein, refers to a chemical compound that alone or in combination with other compounds can be used to aid the uptake of a further substance across an epithelium or other biological membrane. In particular, the term "permeation enhancer" is used herein to refer to a chemical compound that alone or in combination with other compounds can be used to aid the uptake of a further substance across the buccal mucosa. Permeation enhancers can typically be divided into two different categories, paracellular (para) or transcellular (trans) permeability enhancers, according to their mechanism of action. Paracellular permeation enhancers are those which aid the uptake of a further substance through the intercellular space between the cells in an epithelium or other biological membrane. Transcellular permeation enhancers are those which aid the uptake of a further substance through the cells in an epithelium or other biological membrane, wherein the further substance passes through both the apical and basolateral cell membranes in the epithelium or other biological membrane.

Typically, the film may comprise one or more paracellular permeation enhancers. Alternatively, the film may comprise one or more transcellular permeation enhancers. Alternatively, the film may comprise at least one paracellular permeation enhancer and at least one transcellular permeation enhancer.

Typically, the permeation enhancer, if present, is one or more compounds selected from: non-ionic, cationic, anionic or zwitterionic surfactants (e.g. caprylocaproyl polyoxyl-8 glyceride, sodium lauryl sulfate, cetyltrimetyl ammonium bromide, decyldimethyl ammonio propane sulfonate); bile salts (e.g. sodium deoxycholate); fatty acids (e.g. hexanoic acid, hetptanoic acid, oleic acid); fatty amines; fatty ureas; fatty acid esters (e.g. methyl laurate, methyl palmitate); substituted or unsubsituted nitrogen-containing heterocyclic compounds (e.g. methyl pyrrolidone, methyl piperazine, azone); terpenes (e.g. limonene, fenchone, menthone, cineole); sulfoxides (e.g. dimethylsulfoxide, DMSO); ethylenediaminetetraacetic acid (EDTA); and combinations thereof. Preferably, the permeation enhancer, if present, is selected from EDTA, oleic acid, and combinations thereof.

Typically, the film may comprise EDTA. Without wishing to be bound by any particular theory, EDTA is believed to act as a paracellular permeation enhancer by transiently affecting tight junctions interconnecting membrane cells, and subsequently increasing paracellular or pore transport. EDTA is also believed to act as a transcellular permeation enhancer by interaction with phospholipid headgroups and increasing membrane fluidity [4]. Alternatively, the film may comprise oleic acid. Without wishing to be bound by any particular theory, oleic acid is believed to act as a transcellular permeation enhancer by interacting with the polar head groups of phospholipids in or on cell membranes, and increasing cell membrane flexibility, thereby promoting transcellular drug permeability. Oleic acid has been shown to demonstrate enhanced permeability with porcine buccal epithelium at a concentration of 1-10% [5].

The film may additionally comprise a self-emulsifying drug delivery system (SEDDS) or resulting emulsion thereof. Such a system may preferably be a self-microemulsifying drug delivery system (SMEDDS) or resulting emulsion thereof or a self-nanoemulsifying drug delivery system (SNEDDS) or resulting emulsion thereof. Self-microemulsifying drug delivery systems are microemulsion preconcentrates or anhydrous forms of microemulsion. Self-nanoemulsifying drug delivery systems are nanoemulsion preconcentrates or anhydrous forms of nanoemulsion. These systems are typically anhydrous isotropic mixtures of oil (e.g. tri-, di- or mono- glycerides or mixtures thereof) and at least one surfactant (e.g. Span®, Tween®), which, when introduced into aqueous phase under conditions of gentle agitation, spontaneously form an oil-in-water (O/W) microemulsion or nanoemulsion (respectively). SNEDDS systems typically form an emulsion with a globule size less than 200 nm [6]. SEDDS (e.g. SMEDDS or SNEDDS) may also contain coemulsifier or cosurfactant and/or solubilizer in order to facilitate emulsification (e.g. micoremulsification or nanoemulsification) or improve the drug incorporation into the SEDDS (e.g. SMEDDS or SNEDDS). Typically, the SEDDS (e.g. SMEDDS or SNEDDS) components is selected from: a mixture of Tween® with one or more glycerides and a hydrophilic cosolvent; a mixture of Tween® with a low HLB cosurfactant and a hydrophilic cosolvent; a mixture of a polyethyleneglycol (PEG), Labrasol and Chremophore EL; a mixture of polyethyleneglycol (PEG), Labrasol and Kolliphore EL; and a mixture of polyethyleneglycol (PEG), Labrasol, Chremophore EL and Chremophore RH40. The PEG may be any suitable polyethyleneglycol such as PEG with an average molecular weight of from 100 to >1000 Da, preferably from 200 to 800 Da, more preferably from 300 to 600 Da, and most preferably about 400.

The term "glyceride", as defined herein, refers to any ester formed between glycerol and one or more fatty

acids. The term "glyceride" may be used interchangeably with the term "acylglycerol". Typically, the glyceride is a monoglyceride, a diglyceride or a triglyceride. Preferably, the glyceride is a triglyceride. Typically, the glyceride is a simple glyceride. The term "simple glyceride" refers to a diglyceride in which the two fatty acids are the same as one another, or a triglyceride in which the three fatty acids are the same as one another. Alternatively, the glyceride is a mixed glyceride. The term "mixed glyceride" refers to a diglyceride in which the two fatty acids are different one another, or a triglyceride in which either one of the three fatty acids is different to the other two, or all three of the fatty acids are different to one another. Therefore, the glyceride is typically a monoglyceride, a simple diglyceride, a simple triglyceride, a mixed diglyceride, or a mixed triglyceride. Preferably, the glyceride is a simple triglyceride or a mixed triglyceride.

A "hydrophilic cosolvent", as defined herein, is any solvent that is miscible with water. Examples of suitable hydrophilic cosolvents include, but are not limited to: glycerol, ethanol, 2-(2-ethoxyethoxyethanol), PEG-400 and propylene glycol.

The term "low HLB cosurfactant", as defined herein, refers to any lipid falling within class IIIA, IIIB or IV of the lipid formulation classification system described by C.W. Pouton [7].

Typically, the film may additionally comprise any suitable chelating agent. A chelating agent may be added to the film to act as a preservative. A "chelating agent", as defined herein, refers to a chemical compound that is a multidentate ligand that is capable of forming two or more separate bonds to a single central atom, typically a metal ion. Examples of suitable chelating agents include, but are not limited to: ethylenediaminetetraacetic acid (EDTA), ethylene glycol-bis(β-aminoethyl ether)-*N*,*N*,*N'*,*N'*-tetraacetic acid (EGTA), 1,2-bis(*ortho*-aminophenoxy)ethane-*N*,*N*,*N'*,*N'*-tetraacetic acid (BAPTA), citric acid, phosphonic acid, glutamic acid, histidine, malate, and derivatives thereof. Preferably, the chelating agent, if present, is ethylenediaminetetraacetic acid (EDTA). The film may comprise from 0.001% to 4% by weight of each chelating agent present. Preferably, the film may comprise from 0.001% to 0.1% by weight of each chelating agent present.

The film may additionally comprise any suitable antioxidant. An "antioxidant", as defined herein, is any compound that inhibits the oxidation of other chemical species. Examples of suitable antioxidants include, but are not limited to: ascorbic acid; citric acid; sodium bisulfite; sodium metabisulfite; ethylenediaminetetraacetic acid (EDTA); butyl hydroxitoluene; and combinations thereof. Preferably, the antioxidant, if present, is ascorbic acid, sodium bisulfite, or a combination thereof. More preferably, the antioxidant, if present, is ascorbic acid. Most preferably, both ascorbic acid and sodium bisulfite are present as antioxidants. Preferably, the film may comprise from 0.001% to 4% by weight of each antioxidant present, more preferably from 0.001% to 0.1% by weight of each antioxidant present.

Typically, the film may additionally comprise any suitable antimicrobial agent. An "antimicrobial agent", as defined herein, is any compound that kills microorganisms or prevents their growth. Examples of suitable antimicrobial agents include, but are not limited to: benzyl alcohol; benzalkonium chloride; benzoic acid; methyl-, ethyl- or propylparaben; and quarternary ammonium compounds. The film may comprise from 0.001% to 4% by weight of each antimicrobial agent present. Preferably, the film may comprise from 0.001% to 0.1% by weight of each antimicrobial agent present.

EDTA may therefore be present in a film according to the present invention as an antioxidant, as a permeation enhancer or as a chelating agent. Typically, if EDTA is present, the EDTA acts as all of an antioxidant, a permeation enhancer and a chelating agent. Alternatively, if EDTA is present, the EDTA may act only as an antioxidant. Alternatively, if EDTA is present, the EDTA may act only as a permeation enhancer. Alternatively, if EDTA is present, the EDTA may act only as a chelating agent.

Optionally, the film may additionally comprise at least one inorganic salt. Said inorganic salt may be any salt acceptable for use in the preparation of a medicament. Examples of such salts include, but are not limited to, the halides, oxides, hydroxides, sulfates, carbonates, phosphates, nitrates, acetates and oxamates of the alkali metals, alkaline earth metals, aluminium, zinc and ammonium. Typically, said inorganic salt may be selected from sodium chloride, potassium chloride, magnesium chloride, calcium chloride, and ammonium chloride. Preferably, the inorganic salt is sodium chloride. Typically, the inorganic salt is present in the film in a total concentration of at least 0.05 wt%, preferably in a concentration of from 0.1 to 5 wt%, more preferably from 0.2 to 2 wt%, yet more preferably from 0.25 to 1 wt%, and most preferably about 0.5 wt%. Alternatively, the film does not comprise any inorganic salt. In such an embodiment, the film typically comprises the neutral (i.e. unionized) form of the API.

Typically, the film may additionally comprise at least one excipient, optionally at least one basifying agent or acidifying agent, optionally at least one permeation enhancer, optionally at least one pharmaceutically acceptable solvent, optionally at least one buffering component, optionally at least one antioxidant, and optionally a SEDDS (e.g. SMEDDS or SNEDDS). For example, the film may comprise at least one excipient, at least one basifying agent or acidifying agent, optionally at least one permeation enhancer, optionally at least one anitoxidant and optionally at least one buffering component. Preferably, the film may comprise glycerol, sorbitol, optionally at least one basifying agent or acidifying agent, optionally at least one permeation enhancer, optionally at least one antioxidant, and optionally at least one buffering component. More preferably, the film may comprise glycerol, sorbitol, xylitol, and optionally at least one basifying agent. Even more preferably, the film may comprise glycerol, sorbitol, xylitol and sodium hydroxide.

Preferably, the film according to the present invention comprises from 15% to 99% by weight of the alginate salt of a monovalent cation or the mixture of alginate salts containing at least one alginate salt of a monovalent cation, from 0% to 20% by weight of water, from 0.001% to 75% by weight of the API, from 0% to 40% by weight of glycerol, from 0% to 40% by weight of sorbitol, optionally from 0% to 40% by weight of xylitol, optionally a basifying agent or an acidifying agent, optionally from 0.01% to 5% by weight of a permeation enhancer, optionally from 0.01% to 10% by weight of at least one antioxidant, optionally from 0.1% to 10% by weight of a SEDDS (e.g. SMEDDS or SNEDDS), and optionally from 0.001% to 4% by weight of a chelating agent. More preferably, the film according to the present invention comprises from 25% to 91% by weight of the alginate salt of a monovalent cation or the mixture of alginate salts containing at least one alginate salt of a monovalent cation, from 9% to 11% by weight of water, from 0.2% to 45% by weight of the API, from 5% to 20% by weight of glycerol, from 5% to 20% by weight of sorbitol, from 5% to 20% by weight of xylitol, and optionally a basifying agent or an acidifying agent.

Alternatively, the film according to the present invention consists of from 15% to 99% by weight of the alginate salt of a monovalent cation or the mixture of alginate salts containing at least one alginate salt of a monovalent cation, from 0% to 20% by weight of water, from 0.001% to 75% by weight of the API, from 0% to 40% by weight of glycerol, from 0% to 40% by weight of sorbitol, optionally from 0% to 40% by weight of xylitol, optionally a basifying agent or an acidifying agent, optionally from 0.01% to 5% by weight of a permeation enhancer, optionally from 0.01% to 10% by weight of at least one antioxidant, optionally from 0.1% to 10% by weight of a SEDDS (e.g. SMEDDS or SNEDDS), and optionally from 0.001% to 4% by weight of a chelating agent. More preferably, the film according to the present invention consists of from 25% to 91% by weight of the alginate salt of a monovalent cation or the mixture of alginate salts containing at least one alginate salt of a monovalent cation, from 9% to 11% by weight of water, from 0.2% to 45% by weight of the API, from 5% to 20% by weight of glycerol, from 5% to 20% by weight of sorbitol, from 5% to 20% by weight of xylitol, and optionally a basifying agent or an acidifying agent.

A film according to the invention preferably has a thickness before drying of 200 to 2000 μm, more preferably from 300 to 1750 μm, even more preferably from 400 to 1500 μm, and most preferably from 1000 to 1500 μm.

A film according to the invention preferably has a surface area on each of its two largest faces of from 0.1 to 20 $cm^2$, more preferably from 0.5 to 15 $cm^2$, even more preferably from 1 to 10 $cm^2$ and most preferably from 2 to 6 $cm^2$. Preferably, the surface area of each of the two largest faces of the film is about 3 $cm^2$ or about 5 $cm^2$.

The skilled person, having regard for the desired time of dissolution for a given application, will be able to select a suitable film thickness and surface area by simply preparing films of a range of different thicknesses and surface areas and testing the resultant films to measure the dissolution time.

The mechanical properties of a film according to the invention are very satisfactory. In particular, the film is flexible (i.e. it permits bending and folding without breaking), and has a high tensile strength. Importantly, the film of the present invention is not a gel, since the alginate polymer strands are not cross-linked with one another. The film of the invention is bioadhesive; that is to say that the film comprises a natural polymeric material (alginate) which can act as an adhesive. The film is adhesive to moist surfaces, such as mucosa. In particular, the film is adhesive to mucosa of the oral cavity, such as mucosa in the buccal, labial, sublingual, ginigival or lip areas, the soft palate and the hard palate.

The film according to the invention may be provided with printed text matter or printed images thereon, e.g. a brand name, a trade mark, a dosage indication or a symbol.

*Administration and uses of the films in treatment*

In general, films of the present invention are administered to a human patient so as to deliver to the patient a therapeutically effective amount of the active pharmaceutical ingredient (API), preferably ketamine or a pharmaceutically acceptable salt thereof, contained therein.

As used herein, the term "therapeutically effective amount" refers to an amount of the API which is sufficient to reduce or ameliorate the severity, duration, progression, or onset of a disorder being treated, prevent the advancement of a disorder being treated, cause the regression of, prevent the recurrence, development, onset or progression of a symptom associated with a disorder being treated, or enhance or improve the prophylactic or therapeutic effect(s) of another therapy. The precise amount of API administered to a patient will depend on the type and severity of the disease or condition and on the characteristics of the patient, such as general health, age, sex, body weight and tolerance to drugs. It will also depend on the degree, severity and type of the disorder being treated. The skilled artisan will be able to determine appropriate dosages depending on these and other factors.

As used herein, the terms "treat", "treatment" and "treating" refer to the reduction or amelioration of the progression, severity and/or duration of a disorder being treated, or the amelioration of one or more symptoms (preferably, one or more discernible symptoms) of a disorder being treated resulting from the administration of a film according to the invention to a patient.

Typically, a film according to the present invention is provided for use in the treatment of a human patient. Typically, the film according to the invention is provided for use in anesthesia, pain management, or the treatment of a

condition selected from amnesia, depression and bipolar disorder, in a human patient.

Preferably, the film according to the invention is provided for use in anesthesia. Anesthesia is a state of controlled, temporary loss of sensation or awareness that is induced for medical purposes. It may include analgesia (relief from or prevention of pain), paralysis (muscle relaxation), amnesia (loss of memory) or unconsciousness. In particular, anesthesia enables the painless performance of medical procedure that would otherwise cause severe or intolerable pain to an unanesthetized pateient, or would otherwise be technically unfeasible. Anesthesia includes general anesthesia (suppression of central nervous system activity, resulting in unconsciousness and total lack of sensation), sedation (suppressing the central nervous system to a lower degree, limiting both anxiety and the creation of long-term memories without resulting in unconsciousness) and regional or local anesthesia (blocking of transmission of nerve impulses from a specific part of the body). Thus, the film according to the invention is typically provided for use in general anesthesia, sedation and/or regional or local anesthesia. Typically, the film according to the invention is provided for use during surgery performed on a human patient.

The present invention provides a film according to the invention for use in the treatment of amnesia. Amnesia is a deficit in memory caused by brain damage or disease, but it can also be caused temporarily by the use of various sedatives and hypnotic drugs. The memory can be either wholly or partially lost due to the extent of damage caused. Amnesia includes retrograde amnesia (the inability to retrieve information that was acquired before a particular date) and anterograde amnesia (the inability to transfer new information from the short-term memory store into the long-term memory store). Amnesia is typically associated with damage to the medial temporal lobe. Other areas, such as the hippocampus, are also involved with memory. Thus, the film according to the invention may be provided for use in the treatment of retrograde and/or anterograde amnesia.

The present invention provides a film according to the invention for use in the treatment of depression. Depression is a state of low mood and aversion to activity. It can affect a person's thoughts, behaviour, motivation, feelings, and sense of well-being. Symptoms of depression include sadness, difficulty in thinking and concentration and a significant increase/decrease in appetite and time spent sleeping, and feelings of dejection, hopelessness and, sometimes, suicidal feelings. It can be either short-term or long-term. Depressed mood may be a symptom of certain mood disorders such as major depressive disorder or dysthymia. Thus, the film according to the invention may be provided for use in the treatment of major depressive disorder or dysthymia. The film according to the invention may be provided for use in the treatment or amelioration of symptoms selected from sadness, difficulty in thinking and concentration, low mood, aversion to activity, increased appetite, loss of appetite, insomnia, feelings of dejection, feelings of hopelessness and suicidal thoughts.

The present invention provides a film according to the invention for use in the treatment of bipolar disorder. Bipolar disorder is a chronic, recurrent, severe, and often debilitating illness characterised by one or more episodes of mania, depression and long-term psychosocial disability. Bipolar disorders in general include bipolar disorder and unstable bipolar disorder with rapid fluctuations (rapid cyclers), manic-depressive disorders, acute mania, mood episodes, and manic and hypomanic episodes. A manic episode is a period of abnormally elevated mood, accompanied by abnormal behaviour that disrupts life, and includes, for example, flying suddenly from one idea to the next; rapid, "pressured" and loud speech; increased energy, with hyperactivity and a decreased need for sleep; inflated self-image; excessive spending; hypersexuality; and/or substance abuse. Elevated mood can manifest itself as either euphoria or as irritability. Thus, the film according to the invention may be provided for use in the treatment of bipolar disorder and unstable bipolar disorder with rapid fluctuations (rapid cyclers), manic-depressive disorders, acute mania, mood episodes, or manic and/or hypomanic episodes. The film according to the invention may be provided for use in the treatment or amelioration of symptoms selected from one or more episodes of mania, depression, long-term psychosocial disability, and abnormally elevated mood accompanied by abnormal behaviour.

Typically, the patient to be treated is an adult. Alternatively, the patient to be treated may be a child. The patient to be treated may be an elderly patient. The patient to be treated may be a child suffering from allergies.

Typically, the film is administered to the oral cavity of the patient. The film is preferably applied to an oral mucosa in the buccal or labial or sublingual areas or to the soft palate. The film is typically applied by the patient themselves. Alternatively, the film is administered to the patient by another person, e.g. a medical practitioner, a nurse, a carer, a social worker, a colleague of the patient or a family member of the patient.

The film is bioadhesive and adheres to the surface of the oral cavity upon application. After application, the alginate film begins to dissolve, releasing the active pharmaceutical ingredient. Typically, the film fully dissolves in a time period of from 0.1 to 60 minutes or more after application to the mucosa of the oral cavity. Preferably, the film fully dissolves in a time period of from 0.5 to 30 minutes, more preferably from 1 to 20 minutes, still more preferably from 3 to 10 minutes, and most preferably from 3 to 5 minutes after application to the mucosa of the oral cavity.

Without wishing to be bound by any particular theory, it is believed that as the film dissolves within the oral cavity, the active pharmaceutical ingredient which is concomitantly released may enter the bloodstream by one or both of two different routes: (a) *via* absorption across the oral mucosa directly into the bloodstream (the "oral transmucosal route"); and (b) *via* swallowing into the stomach and subsequent absorption across the epithelium of the intestines into

the bloodstream. Typically the peak plasma concentration of the API in a patient exceeds 1 ng/mL. This peak plasma concentration may be achieved within 120 minutes from adhesion of the film to the mucosa of the oral cavity, preferably within 60 minutes from adhesion, more preferably within 45 minutes, even more preferably within 30 minutes or 20 minutes from adhesion, and most preferably within 10 minutes from adhesion.

Typically, a single film is applied to the patient, generally to the mucosa of the oral cavity, at a given time. However, in some cases it may be desirable to apply two films simultaneously to achieve the correct dose for an individual patient. When the API is ketamine, and it is being used for anesthesia or to treat acute pain, the recommended dosage for adults is 20-80mg. When used to treat acute depression, the recommended dose ranges from 10mg to 100mg. In some cases it may be desirable to apply more than two films simultaneously to achieve the correct dose for an individual patient, for example, three, four, five, six, seven, eight, nine, ten or more.

The present invention also provides a product comprising one or more films according to the invention, and packaging. Each of the films may individually be wrapped within a pouch, or multiple films may be wrapped together within the same pouch. Optionally, said pouch is made from PET-lined aluminium. The product may further comprise instructions for use of the film. These instructions may contain information on the recommended frequency or timing of use of the film by a patient, how to use remove the film from its pouch or packaging, how to adhere the film to a mucous membrane, and where within the oral cavity to adhere the film to a mucous membrane.

Any film or films of the present invention may also be used in combination with one or more other drugs or pharmaceutical compositions in the treatment of disease or conditions for which the films of the present invention and/or the other drugs or pharmaceutical compositions may have utility.

The one or more other drugs or pharmaceutical compositions may be administered to the patient by any one or more of the following routes: oral, systemic (e.g. transdermal, intranasal, transmucosal or by suppository), or parenteral (e.g. intramuscular, intravenous or subcutaneous). Compositions of the one or more other drugs or pharmaceutical compositions can take the form of tablets, pills, capsules, semisolids, powders, sustained release formulations, solutions, suspensions, elixirs, aerosols, transdermal patches, bioadhesive films, or any other appropriate compositions. The choice of formulation depends on various factors such as the mode of drug administration (e.g. for oral administration, formulations in the form of tablets, pills or capsules are preferred) and the bioavailability of the drug substance.

*Manufacture of the films*

The films according to the invention may be manufactured by preparing a film-forming solution by addition and mixing of the constituent components of the film, distributing this solution onto a solid surface, and permitting the solution to dry on the surface to form a film. To distribute a solution or composition onto a solid surface the solution or composition may simply be poured onto and/or spread evenly over the surface, e.g. by use of a draw-down blade or similar equipment.

A typical method includes the process steps of:

(a) mixing the API in water;
(b) optionally, subsequently adjusting the pH of the solution to the desired level by addition of an appropriate acid or base, typically a concentrated acid, and preferably adjusting the pH of the solution to from 2 to 4;
(c) optionally, adding one or more additives selected from xylitol, a cyclodextrin, poly(vinyl pyrrolidone), hydroxypropylmethylcellulose, poly(acrylic acid) and pullulan to the solution;
(d) optionally, mixing one or more excipients into the solution;
(e) adding the alginate salt of monovalent cation under suitable conditions to result in the formation of a viscous cast;
(f) optionally, adding further water to the cast;
(g) optionally, sonicating the cast;
(h) leaving the cast to de-aerate;
(i) pouring the cast onto a surface and spreading the cast out to the desired thickness;
(j) drying the cast layer, typically at a temperature of from 30 to 70 °C until the residual water content of the film is from 0 to 20% by weight and a solid film is formed; and
(k) optionally, cutting the solid film into pieces of the desired size, further optionally placing these pieces into pouches, preferably wherein the pouches are made from PET-lined aluminium, sealing the pouches and further optionally, labelling them.

Accordingly, a typical method of manufacturing a film comprising a neutral compound of Formula (I) and an acid $H_xA$ as defined herein is as follows:

(a) mixing the API in water, and subsequently adjusting the pH of the solution to the desired level by addition of an appropriate acid or base, typically a concentrated acid, and preferably adjusting the pH of the solution to from 2 to 4;
(b) optionally, mixing one or more excipients into the solution;

(c) adding the alginate salt of monovalent cation under suitable conditions to result in the formation of a viscous cast;
(d) adjusting the pH of the solution to the desired level by addition of an appropriate acid or base, typically a diluted acid or alkali, preferably a diluted alkali, and preferably adjusting the pH of the solution to from 3 to 5;
(e) optionally, sonicating the cast;
(f) leaving the cast to de-aerate;
(g) pouring the cast onto a surface and spreading the cast out to the desired thickness;
(h) drying the cast layer, typically at a temperature of from 30 to 70 °C until the residual water content of the film is from 0 to 20% by weight and a solid film is formed; and
(i) optionally, cutting the solid film into pieces of the desired size, further optionally placing these pieces into pouches, preferably wherein the pouches are made from PET-lined aluminium, sealing the pouches and further optionally, labelling them.

A typical method of manufacturing a film comprising a pharmaceutically acceptable salt of a compound of Formula (I) and an additive as defined herein is as follows:

(a) mixing the salt of the API in water;
(b) adding one or more additives selected from xylitol, a cyclodextrin, poly(vinyl pyrrolidone), hydroxypropylmethylcellulose, poly(acrylic acid) and pullulan to the solution;
(c) optionally, mixing one or more excipients into the solution;
(d) adding the alginate salt of monovalent cation under suitable conditions to result in the formation of a viscous cast;
(e) optionally, adding further water to the cast;
(f) optionally, sonicating the cast;
(g) leaving the cast to de-aerate;
(h) pouring the cast onto a surface and spreading the cast out to the desired thickness;
(i) drying the cast layer, typically at a temperature of from 30 to 70 °C until the residual water content of the film is from 0 to 20% by weight and a solid film is formed; and
(j) optionally, cutting the solid film into pieces of the desired size, further optionally placing these pieces into pouches, preferably wherein the pouches are made from PET-lined aluminium, sealing the pouches and further optionally, labelling them.

In an alternative variant of any of the above methods, after the viscous cast is poured onto a surface, it is first spread out to a thickness of about 2 mm by means of an applicator with a slit height of about 2 mm, and is then subsequently spread out to a thickness of about 1 mm by means of an applicator with a slit height of about 1 mm.
Typically, the alginate salt(s) are added to the API-containing water solution. Alternatively, the API and the alginate salt(s) are both dissolved together in solution. Alternatively, the API may be added to the alginate solution so as to give an emulsion or suspension of the API in the alginate solution. Alternatively, the film-forming composition of the invention may comprise both dissolved and non-dissolved active ingredients. For example, a film-forming composition may comprise a combination of active ingredient dissolved in the alginate solution and active ingredient suspended in the solution.
Additional API may be applied to the surface of the film before or after drying, e.g. as an aerosol spray onto a dry or wet film. An active ingredient may also be applied as a powder onto the surface of the film. A flavouring agent may additionally be applied in such a way.
Herein, any reference to a term in the singular also encompasses its plural. Where the term "comprising", "comprise" or "comprises" is used, said term may substituted by "consisting of', "consist of" or "consists of" respectively, or by "consisting essentially of", "consist essentially of' or "consists essentially of" respectively. Any reference to a numerical range or single numerical value also includes values that are about that range or single value. Any reference to alginate encompasses any physiologically acceptable salt thereof unless otherwise indicated. Unless otherwise indicated, any % value is based on the relative weight of the component or components in question.

## Examples

The following are Examples that illustrate the present invention. However, these Examples are in no way intended to limit the scope of the invention. References to "ketamine" or a pharmaceutically acceptable salt thereof throughout this Examples section refer to a racemic mixture of ketamine enantiomers unless specified otherwise. Only examples comprising either (i) an acid $H_xA$, wherein A is a counterion having an ionic radius of 2.65 Å or greater, and x is a positive integer which is equal to the charge of the counterion A, or (ii) an additive selected from xylitol, a cyclodextrin, poly(vinyl pyrrolidone), hydroxypropylmethylcellulose, poly(acrylic acid) and pullulan, are according to the claims.

**Example 1: Preparation of films comprising ketamine hydrochloride as active agent**

First attempts (described below) to produce oral films containing ketamine hydrochloride as active agent resulted in the presence of crystals in the final film products.

***Protocol for film preparation***

Ketamine hydrochloride films were prepared using the initial batch formulas of two dose strengths (5 mg and 1 mg) as listed in Table 1.

*Table 1. Batch formulae for 5 mg and 1 mg initial ketamine.HCl buccal films.*

| Ingredient | Amount in 5 mg dose strength batch | Amount in 1 mg dose strength batch | Function |
|---|---|---|---|
| Ketamine.HCl | 3 g | 0.6 g | API |
| Glycerol | 3 g | 3 g | Plasticizer |
| Sorbitol | 3.5 g | 3.5g | Plasticizer |
| Xylitol | 5 g | 5g | Plasticizer |
| Water | 100 mL | 100 mL | Solvent |
| Sodium alginate (Protanal 5/60) | 13.35 g | 13.35g | Film-forming polymer |

Xylitol was added to the formulation to improve the pliability of the ketamine films. The pH of the solution, before being mixed with alginate was 3.5, indicating that ketamine hydrochloride is an acidic salt. However, the pH of the cast, i.e. after addition of alginate, was pH 5.5 and therefore is a suitable pH for application to the oral mucosa, without any further pH adjustment. The films were produced according to the following procedure:

- Ketamine hydrochloride was dissolved in the majority of the purified water under mixing, followed by the addition of glycerol/sorbitol/xylitol.
- The batch volume was increased to the correct total amount by addition of the remainder of the purified water.
- The sodium alginate was added under mixing for about 30 minutes or until a lump free dispersion was achieved, resulting in a viscous cast.
- The cast was left overnight for de-aeration.
- The cast was poured onto a glass plate and spread out to a thickness of 1 mm by means of an applicator.
- The cast layer was dried in a drying cabinet heated to approximately 40 °C until a residual water content of about 10% by weight was achieved and a solid film was formed.
- The solid film was cut into pieces measuring 15 x 20 mm with a knife.
- The resulting films were placed individually into aluminium/polyethylene terephthalate (PET) pouches, sealed with a heat sealer and labelled.

Fresh ketamine 5 mg films were found to be transparent, and no crystals/flocs were found when analysed under a light microscope. After a few hours, however, round flowery flocs were observed in the film, which spread with time and turned into a lining-pattern in the overall film. In ketamine films produced at lower dose strength (1 mg/dose), some small shiny structures also appeared in the film after 50 hrs in a time-dependent microscopy study.

These tiny structures or flocs can be either solid crystals or solid amorphous ketamine hydrochloride or a molecular dispersion. It is known that molecular dispersion should not show up as individual particles under a light microscope and that solid amorphous particles appear as non-shiny particles or flocs. Dissolution tests were performed with a fresh ketamine 5 mg film and 3 months old ketamine 5 mg film in order to determine the origin of the flocs/shiny structures. A placebo film was taken as the control sample in the dissolution test. The dissolution experiment was carried out using a 50 mL beaker containing 20 mL milliQ water (room temperature), stirred with a magnetic stirrer (size 15 mm x 6 mm) at 300 rpm. The test ketamine film was stick to the wall of glass beaker, mimicking the *in vivo* condition, i.e. sticking of the film to the oral mucosa. Samples were collected at different time intervals and the released ketamine in the dissolution media was analysed by reverse-phase HPLC. The dissolution time of film was determined visually. Fresh ketamine 5 mg film as well as the placebo film were dissolved in 180 s, whereas it took over 350 s for complete dissolution of the old ketamine 5 mg film. The results are shown in Fig. 1. The fact that the old ketamine 5 mg film containing flocs

structures took longer to dissolve than the fresh ketamine 5 mg film containing flocs structures confirmed that the flocs structures present in ketamine film are crystals or crystal lumps, as opposed to an amorphous structure or molecular dispersion (which would be anticipated to have the same dissolution time for both old and fresh films).

Thus, ketamine hydrochloride films produced via this method were found not to be optimal preparations, even at a low dose strength (1 mg).

***Strategies to overcome the crystal growth problem***

One strategy formulated to improve the stability of films containing ketamine hydrochloride was to include a component in the film which might act as a crystal growth inhibitor. In this regard, different polymers, additional plasticizer, and solvents were included in the basic formulation to test for any effect on crystal growth inhibition. The tested polymers are set out in Table 2 below. Further, the effect of various factors such as drug:polymer ratio, drug: plasticizer ratio, and effect of various solvents on inhibiting the crystal growth in the film was also studied.

*Table 2. Different types of materials that were included in the basic formulation to test their effect on inhibiting crystal growth.*

| Material | Category |
|---|---|
| Xylitol | Plasticizer |
| PEG 400 | Solvent/Plasticizer |
| Tween 80 | Surfactant |
| Polyvinyl pyrrolidone (PVP K30) | Polymer |
| Polyacrylic acid | Polymer |

An alternative approach devised is to use free *"ketamine base"* in the film formulations. This eliminates the presence of chloride counterions in the films. *"Ketamine base"* was prepared by precipitating ketamine from its salt (hydrochloride) aqueous solution above its $pK_a$ value of 7.6. For this purpose, concentrated NaOH solution (4 M) was used to maintain the pH between 8-9. A suspension was obtained due to drug precipitation, was filtered through a 0.45 μm pore size filter and washed with dilute NaOH solution (0.001 mM). The resulting powder was ketamine free base, and dried in the desiccator for 3-4 days.

Since ketamine has a secondary amine group ($R-CH_2-NH-CH_3$) that ionizes at lower pH, different weak acids such as ascorbic acid, phosphoric acid, tartaric acid were used to solubilize the ketamine base at an acidic pH of 4. Nitric acid, which is a strong acid not containing chloride ions, was used in a control experiment.

Table 3 below thus summaries the different formulations to be developed and evaluated in an attempt to solve the crystal growth problem in the ketamine hydrochloride containing films.

*Table 3. Formulations to be evaluated in this study.*

| Ketamine hydrochloride + additive | Ketamine base + acidification to pH 4.0 |
|---|---|
| Basic recipe with increased Xylitol concentration | Basic recipe with Ascorbic acid |
| Basic recipe with PEG 400 (5% w/v) | Basic recipe with Polyacrylic acid |
| Basic recipe with Tween 80 (5% w/v) | Basic recipe with Phosphoric acid |
| Basic recipe with Polyvinyl pyrrolidone (PVP K30) | Basic recipe with Nitric acid |
| Basic recipe with Polyacrylic acid | Basic recipe with Hydrochloric acid |

***Physical evaluation criteria***

After manufacture, each of the batches of ketamine-containing films are evaluated with respect to the following criteria:

| Property | Criteria |
|---|---|
| 1. Cast texture: | lump free, homogenous viscous cast (visual inspection) free of bubbles prior to coating (visual inspection) |

(continued)

| Property | Criteria |
|---|---|
| 2. Residual moisture*: | 9-11% (in process control) |
| 3. Film appearance**: | - translucent and colour homogenous (visual inspection)<br>- smooth and flat surface structure (visual inspection)<br>- pliable and flexible (visual inspection) |
| 4. Dose weight homogeneity: | weighing of doses randomly selected within a film batch |
| 5. Ketamine content***: | RP-HPLC analysis on the changes of dose strengths after stability studies (target dose strength within ±12%) |
| 6. Physical stability: | crystal-free film (optical microscope study) |

* *Residual moisture:* IR-induced water vaporization combined with real-time weight measurement was used. Percentage of change in weight at start until no further change was observed as the measure of residual moisture. [8]

** *Film appearance:* Some film batches were inspected and analyzed with respect to surface structure with light microscope.

*** *Ketamine content and homogeneity:* Reverse phase high-performance liquid chromatography (RP-HPLC) separation with detection at 269 nm was used. Amount of ketamine/dose was calculated using a ketamine standard curve. [9]

**Example 2: Preparation of films comprising ketamine hydrochloride and a putative crystal growth inhibitor**

Batch formulae for each individual dose strength of ketamine.HCl films containing a putative crystal growth inhibitor are listed in Table 4 below. Formulations containing polyacrylic acid were also formulated at 10 mg/dose in addition to 5 mg/dose.

Since PAA based formulations result in increased bubble formation while mixing the alginate in the pre-cast solution, further dilution of cast was required to achieve a less viscous cast and to facilitate removal of air bubbles in the sonication process. For that, an additional 20 mL of milliQ water was added to the final cast resulting in a lower ketamine dose per film (film size $3cm^2$) compared to the standard API dose calculation. To compensate for this, the PAA-based cast was coated at 1.5 mm thickness to obtain the required dose strength.

pH 4 was maintained for formulations containing polyacrylic acid. A pH of 3.5 was obtained when dissolving ketamine.HCl in water. This pH, which is far from the strongest basic pKa of ketamine, was considered appropriate to maintain ketamine in its ionized form at low pH.

The films were produced according to the following procedure:

- Ketamine.HCl was dissolved in the majority of the purified water under mixing, followed by the addition of the putative crystal growth inhibitor.
- The batch volume was increased to the correct total amount by addition of the remainder of the purified water.
- The glycerol and sorbitol were added to the solution under mixing.
- The sodium alginate was added under mixing for about 20 minutes or until a lump free dispersion was achieved, resulting in a viscous cast.
- In the case of a PAA-containing cast, an additional 20 mL of purified water was added.
- The cast was sonicated for 30 minutes.
- The cast was left overnight for de-aeration.
- The cast was poured onto a glass plate and spread out to a thickness of 1 mm by means of an applicator (1.5 mm in the case of PAA-containing casts).
- The cast layer was dried in a drying cabinet heated to approximately 60 °C until a residual water content of about 9-11% by weight was achieved and a solid film was formed.
- The solid film was cut into pieces measuring 15 x 20 mm with a knife.
- The resulting films were placed individually into aluminium/polyethylene terephthalate (PET) pouches, sealed with a heat sealer and labelled.

*Table 4. Batch formulae for two different dose strengths of ketamine.HCl buccal films prepared in the study and containing a putative crystal growth inhibitor. The batch size is about 50 mL, giving a yield of about 250 doses (dose dimension 3cm$^2$). (K/P) represents the ratio of ketamine HCl to polymer on a weight/weight basis. (P/A) represents the amount of PVP to alginate on a weight/weight basis.*

| | **Concentration** | **5 mg/dose formulation** | **10 mg/dose formulation** | **Function of component** |
|---|---|---|---|---|
| | | **(g)** | **(g)** | |
| ***Components of all films*** | | | | |
| Ketamine HCl (g) | | 1.5 | 3.0 | API |
| Sodium alginate (g) | | 6.65 | 6.65 | Film forming agent |
| Sorbitol (g) | | 1.75 | 1.75 | Plasticizer |
| Glycerol (g) | | 1.5 | 1.5 | Plasticizer |
| Xylitol (g) | | 2.5 | 2.5 | Plasticizer |
| Water(g) | | 50 | 50 | Solvent |
| ***Possible crystal growth inhibitors: one of the below present in each film sample*** | | | | |
| Xylitol (g) | Double (2.x) Triple (3.x) | 5 7.5 | - | Plasticizer |
| PEG 400 | 2.5 % | 2.5 | - | Plasticizer |
| Cyclodextrin | 3% | 1.5 | - | |
| Tween 80 | 5% | 2.5 | - | Surfactant |
| PVP K30 | (K/P) ratio, 2:1 w/w<br>(K/P) ratio, 1:2 w/w<br>(P/A) ratio, 1:1 w/w | 0.75<br>3.0<br>6.65 | - | Polymer |
| PAA | (K/P) ratio, 1:2 w/w<br>(K/P) ratio, 1:1.6 w/w<br>(K/P) ratio, 1: 1.1 w/w<br>(K/P) ratio, 1.3:1 w/w | 3.0<br>2.4<br>1.65<br>1.95 | -<br>-<br>4.8<br>3.3 | Polymer |

***Physical evaluation of films***

Ketamine.HCl and all of the putative crystal growth inhibitors were fully dissolved in the liquid (water) phase, and lump free, homogenous (yellowish) viscous casts could be prepared with each individual batch formula/protocol. Viscosity was found to increase with the ketamine content.

Air bubbles generated during preparation of the casts, and which introduce inhomogeneity in the films, were removed by sonicating the cast for a short time and leaving the cast overnight at room temperature for passive de-aeration prior to coating.

All prepared films had smooth, and flat surface structures with flexible properties when dried to a water content of 9-11%. In particular, ketamine films containing PAA as an additive at pH 4 were whitish, homogeneous in appearance, but more opaque.

Quantitative determination of ketamine in the films was performed using RP-HPLC in isocratic mode using FAST analytical method [9] using UV detection at a wavelength of 269 nm. However, stable ketamine formulations at a

higher dose strength (10 mg) were analyzed using a gradient analytical method [9] using UV detection at a wavelength of 210 nm.

The effect of adding each of the different types of putative crystal growth inhibitors to the ketamine film-based formulations are discussed in turn below.

### *(1) Xylitol as putative crystal growth inhibitor*

Xylitol is a well-known sweetener used in pharmaceutical compositions as a taste masking agent. Similar to sorbitol and mannitol, it is a sugar alcohol that can be included in the basic formulation for alginate-based film formulation to improve pliability of drug-loaded films.

Initially, the amount of xylitol present in the "basic" formulation (see Example 1 above) was doubled. In the resulting films, no crystals were observed directly after film preparation; however, large crystal clumps were observed in the film on the $4^{th}$ day after preparation. Compared with the "basic" ketamine 5 mg film described in Example 1, doubling the xylitol concentration in the films resulted in increased stability for the first 2-3 days after preparation. Thus, the onset of crystal formation appeared to have been delayed somewhat by an increase in the xylitol concentration.

Subsequently, the xylitol concentration was further increased to triple the concentration present in the "basic" formulation. Optical microscopy experiments show that this further increase in the xylitol concentration delays the onset of crystal growth until the $6^{th}$ day after film preparation.

Without wishing to be bound by any particular theory, it is hypothesized that xylitol indirectly inhibits crystal growth by dilution of localized ketamine concentration in the film at its increased concentration. Thus, xylitol it thought to disrupt the symmetry of ketamine crystal formation as well as inhibit the nuclei formation in early stage of crystal growth in the film.

### *(2) PEG 400 as putative crystal growth inhibitor*

Glycols, particularly PEG 400, were also considered as possible crystal growth inhibitors. However, in the formulation containing 2.5% (w/v) PEG 400, no inhibitory effect on crystal growth formation was observed in the 5 mg ketamine.HCl film. This could be due to the poor solubility of ketamine.HCl in PEG 400.

### *(3) Cyclodextrin as putative crystal growth inhibitor*

Cyclodextrins (CDs) are a type of cyclic oligosaccharide. They are known to enhance transmucosal drug absorption, most probably by transiently changing membrane permeability, overcoming the diffusion barrier, and opening tight junctions; the greatest enhancement is observed at low concentrations ranging from 2% to 5% w/v. [10], [11] Thus, the trialling of cyclodextrins as crystal growth inhibitors is attractive as it could lead to a dual benefit of enhancing drug absorption and inhibiting crystal growth.

In the present experiment, 3% w/v hydroxypropyl (HP)-beta cyclodextrin was used as an example cyclodextrin in a ketamine 5mg film, to obtain a 1:1 (drug:CD) inclusion complex. After production of the film, no shiny spot was seen in the fresh 5mg ketamine film via optical microscopy, confirming no crystal presence. Crystals did begin to appear in the film on the $8^{th}$ day after production. Therefore, it was concluded that HP betacyclodextrin has some inhibitory effect on crystal formation.

### *(4) Tween 80 (surfactant) as putative crystal growth inhibitor*

Tween 80, also known as Polysorbate 80, is polyoxyethylene sorbitan fatty acid ester. It acts as a nonionic surfactant and is widely used as an emulsifying agent in the preparation of stable oil/water based pharmaceutical emulsions, as a solubilizing agent for a variety of lipophilic substances (including essential oils and oil-soluble vitamins), and as a wetting agent in the formulation of oral and parenteral suspensions at different concentrations.

In the present experiment, Tween 80 was used as a surfactant at a concentration above its critical micelle concentration (CMC). The CMC of Tween 80 in pure water is reported as 0.012 mM. In the formulation, 5% w/v Tween 80 was used in the preparation of a 5 mg ketamine film. Under an optical microscope, crystals were observed as shiny spots in the fresh film. Thus, it was concluded that Tween 80 does not show any significant inhibitory effect on crystal formation in the film.

### *(5) Poly (vinyl pyrrolidone) (PVP K30) as putative crystal growth inhibitor*

PVP consists of vinylpyrrolidone monomer units (as shown in Formula (VI) below) and has wide range of molecular weight from 2500 to 300000. PVP has good solubility in both organic solvent and water. However, its solubility

in aqueous solution is dependent on its molecular weight and size. PVP with a higher molecular weight and longer chain length has lower solubility and produces more viscous solutions upon dissolution.

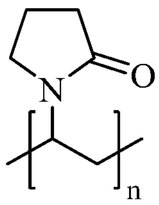

(VI)

Thus, a shorter chain length and low molecular weight PVP variant, PVP K30, was used as representative polymer in the present experiments. Initially, a drug-polymer ratio of 2:1 (w/w) was employed in preparation of a 5 mg ketamine film. Under optical microscopy, crystals began to form after 73 hours. Thus, some delay in nucleation can be observed when adding PVP at this concentration.

Subsequently, the polymer concentration was increased, to a 1:1 (w/w) ratio with the drug. This improved crystal growth inhibition, with the onset of crystal growth delayed until the 7$^{th}$ day after film formation.

A significantly higher concentration of PVP was also tested, whereby PVP K30 was added in a 1:1 (w/w) ratio with the alginate film-forming agent. In this case, no shiny spots were observed by optical microscopy even after storage of the ketamine film for 11 days at room temperature, confirming that crystal formation had been effectively suppressed.

These experiments clearly demonstrate the ability of a film-forming polymer such as PVP to inhibit ketamine.HCl crystal growth. Without wishing to be bound by any particular theory, it is hypothesised that the mechanism of nucleation retardation is mediated by the possible interaction of ketamine with the PVP polymer through hydrogen bonding. Further, crystal growth may be inhibited by the hydrodynamic boundary layer in which the polymer accumulates, as well as by protective layers of polymer adsorbed on the crystal surface.

***(6) Poly(acrylic acid) (PAA) as putative crystal growth inhibitor***

Poly(acrylic acid) (PAA) is soluble in both water and organic solvent. Considering its p$K_a$ of 4.2, PAA is an acidic polymer that consists of acrylic acid monomer (as shown in Formula (VII) below). PAA has a very strong hydrogen bond donor strength and medium hydrogen bond acceptor strength.

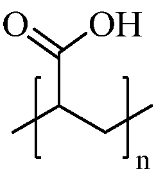

(VII)

Several PAA-containing ketamine casts with different polymer:drug ratios were prepared. The PAA-containing ketamine casts were found to contain air bubbles which were removed via a sonication process before aeration and coating of the cast. The cast was also diluted with additional water for effective sonication to get a bubble-free cast. To compensate for cast dilution, all PAA-containing ketamine films were coated at a film thickness of 1.5 mm to maintain a constant ketamine dose/film.

Optical microscopy experiments showed that no crystals present in 5 mg ketamine film containing a ketamine:PAA (w/w) ratio of 1:2. However, despite effective sonication of the cast, these films were found to contain many air bubbles. Thus formulations having a reduced PAA concentration were prepared. A film containing a ketamine:PAA (w/w) ratio of 1:1.6 showed an absence of crystals in fresh film as well as in a film stored under ambient conditions for 5 days. In addition, this PAA-based 5 mg ketamine film formulation was found to be physically stable (i.e. crystal free) for at least 2 months, after storage in packaging at room temperature.

Since the ketamine 5 mg films were seen to be physically stable at 1:1.6 (w/w) drug:polymer ratio, ketamine films at higher dose strengths were also prepared. For a 10 mg ketamine film containing 1: 1.6 (w/w) drug:polymer ratio, optical microscopy confirmed that the films were crystal free even after 7 days of exposure to moisture at room temperature. In addition, this PAA-based 10 mg ketamine film formulation was found to be crystal free for at least 7 weeks when stored in packaging at room temperature.

Since increased PAA concentration is linked to air bubble formation in the cast, 15 mg ketamine films were

subsequently formulated using the same amount of PAA that was used in the 10 mg ketamine film. Therefore, 15 mg ketamine films were produced at a 1:1.1 (w/w) drug:polymer ratio. The batch formula for these films are shown in Table 5 below.

*Table 5. Batch formula for 15 mg ketamine.HCl buccal films containing PAA as an additive. Films were coated at a thickness of 1.5 mm.*

| Ingredient | Amount | Function |
|---|---|---|
| Ketamine.HCl | 4.5 g | API |
| Water | 70 mL | Solvent |
| Glycerol | 2.5 g | Plasticizer |
| Sorbitol | 1.75 g | Plasticizer |
| Xylitol | 2.5 g | Plasticizer |
| PAA | 5 g | Crystal inhibitor |
| Sodium alginate (Protanal 5/60) | 6.65 g | Film-Forming Polymer |

Optical microscopy confirmed that no crystals appeared in the fresh film or in a film exposed to ambient conditions for 10 days.

Film dose and homogeneity data for the 15 mg films are given in Table 6 below. An acceptable dose variation as well as good homogeneity (mg ketamine/mg film) among the films within the batch were observed.

*Table 6. Dose and homogeneity data for ketamine hydrochloride films containing PAA. RSD = relative standard deviation.*

| PAA based formulation (15mg/dose) (#Batch 28) | |
|---|---|
| *Average dose (mg)* | 16.78 |
| *Standard deviation (mg)* | 2.09 |
| *RSD%* | 12.48 |
| *Ketamine (mg/mg film)* | 175.23 |
| *Number of films analyzed* | 3 |

Formulations with varying drug:polymer ratios (w/w) were also tested to determine a minimum useful PAA concentration which would result in effective crystal growth inhibition. In doing so, 10 mg ketamine films were formulated with a 1.8:1 (w/w) drug:polymer ratio. Large, shiny clumps appeared in the fresh film under polarized view, confirming the presence of crystals, even during the drying process. A further 10 mg ketamine film formulation at 1.3:1 (w/w) drug:polymer ratio also contained crystals in the fresh film. Based on the above formulation results, it was concluded that an excess of PAA over the ketamine active agent is probably required in order to obtain physically stable, crystal free ketamine film formulations.

***Conclusions***

The results from this study demonstrate that it is possible to formulate physically stable ketamine buccal films with ketamine hydrochloride in the presence of substances such as xylitol, cyclodextrins, PVP or polyacrylic acid as an additive. PAA was found to be a particularly effective crystal growth inhibitor and enabled scaling up of production to obtain more concentrated ketamine-containing films. Lab protocols were also developed for these formulations. The main conclusions of the study are summarized below.

- A lump free, homogenous viscous cast, free of bubbles could be obtained by allowing the cast to de-aerate for over 15 hours.
- Films produced were homogenous and had a smooth and flat surface. They were pliable and flexible and easy to handle and considered as being easy to handle and administer for the patient.
- It is possible to formulate crystal-free ketamine films at dose strengths up to approximately 15mg/3cm$^2$ with ketamine

HCl in presence of poly(acrylic acid) at 1:1.1 w/w drug:polymer ratio at pH 4.

- Dose-weight variations obtained in this study were considered fully acceptable for production in lab scale.
- Homogeneity data (mg ketamine/mg film) showed very good consistency within a given batch.

**Example 3: Preparation of films comprising ketamine free base and an acid**

Batch formulae for each individual dose strength of free base ketamine films are listed in Table 7 below. The free base form of ketamine has a secondary amine group ($R-CH_2-NH-CH_3$) which is ionized below pH 7.6. Ketamine films were produced at pH 4 by using different acids including a selection of both strong and weak acids. A ketamine base formulation with hydrochloric acid (HCl) was prepared as a control.

The films were produced according to the following procedure:

- Free ketamine base was dissolved in the majority of the purified water under mixing, followed by the addition of concentrated acid to achieve a pH of approximately 3.0. Stirring was continued for about 1 hour or until a clear solution was obtained.
- The batch volume was increased to the correct total amount by addition of the remainder of the purified water.
- The glycerol and sorbitol (partially dehydrated) were added to the solution under mixing.
- The sodium alginate was added under mixing for about 30 minutes or until a lump free dispersion was achieved, resulting in a viscous cast. The pH was adjusted to pH 4 with a weak acid (e.g. phosphoric acid, malic acid, tartaric acid) and/or a base (e.g. sodium hydroxide).
- The cast was sonicated for 30 minutes.
- The cast was left overnight for de-aeration.
- The cast was poured onto a glass plate and spread out to a thickness of 1 mm by means of an applicator.
- The cast layer was dried in a drying cabinet heated to approximately 50 °C until a residual water content of about 9-11% by weight was achieved and a solid film was formed.
- The solid film was cut into pieces measuring 15 x 20 mm with a knife.
- The resulting films were placed individually into aluminium/polyethylene terephthalate (PET) pouches, sealed with a heat sealer and labelled.

*Table 7. Batch formulae for two different dose strengths of free base ketamine buccal films prepared in the study. The batch size is about 250 doses (dose dimension 3 $cm^2$). q.s. = quantum satis.*

| | **5mg/dose (g)** | **10 mg/dose (g)** | **Component function** |
|---|---|---|---|
| ***Components of all films*** | | | |
| Ketamine base (g) | 1.5 | 3.0 | API |
| Sorbitol (g) | 1.75 | 1.75 | Plasticizer |
| Glycerol (g) | 1.5 | 1.5 | Plasticizer |
| Xylitol (g) | 2.5 | 2.5 | Plasticizer |
| Water (g) | 50 | 50 | Solvent |
| Sodium alginate (g) | 6.65 | 6.65 | Film forming agent |
| ***Possible acids: one of the below present in each film sample*** | | | |
| Ascorbic acid | q.s. to pH 4.0 | q.s. to pH 4.0 | |
| Polyacrylic acid | q.s. to pH 4.0 | q.s. to pH 4.0 | |
| Phosphoric acid | q.s. to pH 4.0 | q.s. to pH 4.0 | |
| Hydrochloric acid | q.s. to pH 4.0 | q.s. to pH 4.0 | |
| Nitric acid | q.s. to pH 4.0 | q.s. to pH 4.0 | |

The effect of adding each of the different types of acid/counterion to the ketamine film-based formulations are discussed in turn below.

*(1) Films containing ascorbic acid*

Films with ketamine base at 5 mg dose strength containing ascorbic acid were produced at pH 4. Optical microscopy confirmed that there were no clearly district shiny spots present in the fresh film, as well as film exposed to moisture for 5 days at room temperature under polarized view. However, some shiny background was observed that might be due to presence of small air bubbles. Thus, it was concluded that 5 mg ketamine films containing ascorbic acid were physically stable and crystal free.

Subsequently, 10 mg ketamine films were also formulated at pH 4. Optical microscopy showed that no crystals were present in fresh film and film exposed to ambient conditions for 3 days. These films were found to be physically stable for 7 weeks when stored in packaging at room temperature.

*(2) Films containing poly(acrylic acid) (PAA)*

In Example 2, it was found that PAA is an effective crystal growth inhibitor for ketamine films containing ketamine HCl. PAA was further considered as a possible additive in films containing ketamine free base. Optical microscopy confirmed that no shiny particles were present in the fresh film, confirming crystal free 5 mg films could be produced.

*(3) Films containing phosphoric acid*

10 mg ketamine films containing phosphoric acid as the pH adjuster were observed to be crystal-free under optical microscopy, both as fresh films and also in films exposed to moisture for 12 days at room temperature. This film formulation was physically stable i.e. crystal free after 7 weeks when stored at room temperature.

An optimized batch formulation is provided in Table 8 below, and film dose and homogeneity data are presented in Table 9. Acceptable dose variation as well as good homogeneity (mg ketamine/mg film) among the films within the batch were observed.

Further, ketamine film formulations containing phosphoric acid were prepared at a dose strength of 20 mg. Optical microscopy confirmed that no shiny crystals were observed in freshly prepared film under polarized view. It was also observed that cast viscosity is related to ketamine content in the formulation. Thus, for the films containing 20 mg ketamine, a reduced amount of alginate was used. The optimized batch formula is provided in Table 10 below.

*Table 8. Batch formula for 10 mg free base ketamine buccal films containing phosphoric acid as pH adjustor. Films were coated at a thickness of 1.2 mm.*

| **Ingredient** | **Amount** | **Function** |
|---|---|---|
| Ketamine base | 3.04 g | API |
| Water | 65 mL | Solvent |
| Glycerol | 1.5 g | Plasticizer |
| Sorbitol | 1.75 g | Plasticizer |
| Xylitol | 2.5 g | Plasticizer |
| Sodium alginate (Protanal 5/60) | 6.65 g | Film-Forming Polymer |
| Phosphoric acid (85%) | 1 mL | pH adjustment |
| NaOH, 1M | 0.250 mL | pH adjustment |

*Table 9. Dose and homogeneity data for ketamine films containing phosphoric acid as pH adjustor. RSD = relative standard deviation.*

| **Ketamine base formulation (10mg/dose) (#Batch 10)** | |
|---|---|
| *Average dose (mg)* | 11.07 |
| *Standard deviation (mg)* | 0.70 |
| *RSD%* | 6.3 |
| *Ketamine (mg/mg film)* | 165.62 |
| *Number of films analyzed* | 3 |

*Table 10. Batch formula for 20 mg free base ketamine buccal films containing phosphoric acid as pH adjustor. Films were coated at a thickness of 1 mm.*

| Ingredient | Amount | Function |
|---|---|---|
| Ketamine base | 10.09 g | API |
| Water | 50 mL | Solvent |
| Glycerol | 1.5 g | Plasticizer |
| Sorbitol | 1.75 g | Plasticizer |
| Xylitol | 2.5 g | Plasticizer |
| Sodium alginate (Protanal 5/60) | 5.5 g | Film-Forming Polymer |
| Phosphoric acid (85%) | 3.1 mL | pH adjustment |
| NaOH, 2M | 1.5 mL | pH adjustment |

***(4) Films containing hydrochloric acid (control)***

Ketamine films with ketamine base at 10 mg dose were formulated at pH 4 with hydrochloric acid. The films turned white during the drying process, confirming rapid crystal growth. Optical microscopy confirmed that the freshly prepared films were not transparent under non-polarized view. This control experiment clearly shows that the presence of chloride ions ($Cl^-$) as counterions makes the ketamine formulation physically unstable. As discussed previously, ketamine.HCl films contain shiny crystals even at a lower dose strength of 1 mg/dose.

***(5) Films containing nitric acid***

A 5 mg ketamine film at pH 4 containing nitric acid as the pH adjustor was produced. Optical microscopy confirmed the presence of crystals in a freshly produced film. Thus, this experiment suggests that chloride ions are not the only counterions that cause nucleation and further crystal growth in film.

In summary, ketamine formulations containing counterions from weak acids are physically stable. In particular, formulations with weak acids such as ascorbic acid, PAA and phosphoric acid were crystal free. On the other hand, ketamine formulations with strong acids such as nitric acid and hydrochloric acid were observed to be physically unstable and contained crystals even in freshly prepared films.

Based on the above results, it was concluded that crystal growth in the ketamine film is primarily related to two factors: (a) molecular volume of counterions; and (b) the electron-withdrawing capacity of the counterions. Therefore, the effect of these counterion properties on crystal growth were considered. Table 11 below presents the molecular volume and the corresponding ionic radius of different counterions. These counterions belong to their corresponding strong and weak acids. Without wishing to be bound by any particular theory, it is believed that counterions having a larger molecular volume disrupt the preferential packing geometry of nucleation growth, thus preventing crystal growth in the film. In this case, counterions such as ascorbate, phosphate, tartrate etc. have a larger molecular volume compared to chloride ions present in the unstable ketamine.HCl formulations.

*Table 11. Molecular volume and ionic radius of counterions derived from a selection of strong and weak acids. Molecular volumes are calculated by using the web-based Chemicalize program (https://chemicalize.com/). This employs "Geometrical Descriptors" to calculate geometrical descriptions such as Van der Waals volume and Van der Waals surface area of a molecule by considering 2D and 3D structural conformation (see https://docs.chemaxon.com/display/docs/Geometrical+Descriptors+Plugin#GeometricalDescriptorsPlugin-fig.2). Ionic radii are measured in the solvated state based on X-ray crystallographic data by Shannon [12]. This value can be different for different coordination numbers, and for high and low spin states of the ions. As reference to Pauling's radii, Shannon has used a value of $r_{ion}$ ($O^{2-}$) = 1.4 Å; data using that value are referred to as "effective " ionic radii. The ionic radii of counterions of weak acids are based on maximum projection radius, calculated using the Chemicalize program (https://chemicalize.com/). These methods for calculating molecular volume and ionic radii are generally applicable in the present invention. Thus, ionic radii may be measured in the solvated state based on X-ray crystallographic data. Molecular volumes may be calculated by using the web-based Chemicalize program (https://chemicalize.com/).*

| **Counter ions** | **Molecular volume (van der Waals volume) (Å$^3$)** | **Ionic radius (Å)** |
|---|---|---|
| *Strong acids* | | |
| Fluoride ($F^-$) | - | 1.33 |
| Chloride ($Cl^-$) | 22.45 | 1.81 |
| Bromide ($Br^-$) | 26.52 | 1.96 |
| Iodide ($I^-$) | - | 2.20 |
| Nitrate ($NO_3^-$) | 40.16 | 2.62 |
| Sulfate ($SO_4^{2-}$) | 59.55 | 2.89 |
| *Weak acids* | | |
| Acetate ($CH_3COO^-$) | 53.50 | 2.97 |
| Ascorbate | 138.94 | 5.39 |
| Phosphate | 56.99 | 2.80 |
| Citrate | 147.19 | 4.55 |
| Tartrate | 113.19 | 3.85 |
| Acrylate | 125.63 | 4.64 |
| Iodate | 51.2 | 3.48 |

Together with the counterions size effect, the mechanism of crystal growth in the film may further be explained in terms of degree of electronegativity. Counterions with a high electron-withdrawing capacity (e.g. chloride ions) may interact strongly with the secondary amine group of ketamine, facilitating crystal formation. As evidence for this theory, it was noted that molecular volume of nitrate counterions is comparable to that of phosphate ions, and yet the effect of these counterions on crystal formation was significantly different. Thus, it was understood that electronegativity of counterions could be a determinant characteristic that influences the crystal growth in the ketamine film. Taken together, it can be suggested that the rate of crystal formation in the film is likely related to electronegativity of counterions, possibly in combination with their molecular volume (Van der Waals volume) in the film. It is expected that the rate of crystal growth in ketamine films increases proportionally to the electronegativity of counterions present in the film.

***Conclusions***

The results from this study demonstrate that it is possible to formulate physically stable ketamine buccal films with ketamine free base in the presence of certain counterions. Lab protocols were also developed for these formulations. The main conclusions of the study are summarized below.

- A lump free, homogenous viscous cast, free of bubbles could be obtained by allowing the cast to de-aerate for over 15 hours.

- Films produced were homogenous and had a smooth and flat surface. They were pliable and flexible and easy to handle and considered as being easy to handle and administer for the patient.
- Results from the study suggests that films at pH 4.0 with dose strengths up to approximately 20 mg/3 $cm^2$ ketamine can be produced as apparent molecular dispersion of ketamine with ketamine base in presence of counter ions of weak acids.
- Dose-weight variations obtained in this study were considered fully acceptable for production in lab scale.
- Homogeneity data (mg ketamine/mg film) showed very good consistency within a given batch.

**Example 4: Dog study using ketamine films**

Three example CBD-containing film formulations, as prepared in Example 1 above, were given to adult beagle dogs (n=3). The first formulation (F1) was a 5 mg alginate film derived from the batch formula in Table 1, the second formulation (F2) was two 5 mg films joined together to provide a 10 mg dose, and the third formulation (F3) was two 5 mg alginate films each placed on opposite sides of the buccal mucosa of the dog. The films were administered to each of the dogs in the study groups by placement of a single film on the buccal mucosa of the dog. As a control, a 5 mg dose of ketamine was also administered intravenously to a control group of the dogs (F4). Plasma was withdrawn from each of the two test groups of dogs, and the control group, over a time-course of from 0 to 480 minutes, and the plasma samples analysed for ketamine concentration (expressed as ng ketamine/mL plasma). For a comparison of absolute exposure levels, the 5 mg doses were adjusted to 10 mg.

Dose-adjusted plasma levels over a time period of 480 minutes for each study group F1-F3, and the control group F4, are shown in Fig. 2. A partial time-course study showing only the first 60-minute period is shown in Fig. 3. Pharmacokinetic parameters from the study are shown in Table 12 below.

*Table 12. Summary of dose-adjusted mean pharmacokinetic parameters from ketamine formulations given to adult beagle dogs (n=3). Dose adjustment of F1 and F4 to 10 mg.*

| Formulation | Dose | $AUC_{0-8hr}$ ng/ml*min | $C_{max}$ ng/ml | $T_{max}$ min |
|---|---|---|---|---|
| **F1** | 5 mg | 2697 | 160 | 13 |
| **F2** | 10 mg | 3333 | 143 | 13 |
| **F3** | 2 x 5 mg | 21103 | 2170 | 3 |
| **F4** | 5 mg | 2056 | 180 | 0 |

These studies show that both film formulations that were administered by adhesion to the buccal cavity (F1, F2 and F3) resulted in good exposure, indicating approximately 100% bioavailability of ketamine at 5 mg. The absorption was rapid in all cases, with a low $T_{max}$ value in all dose groups and individual canine subjects. No apparent increase in ketamine exposure was observed when a single 10 mg ketamine film was employed in place of a single 5 mg film. It was hypothesised that this could be due to saturation of the application site with ketamine active agent. In contrast, the use of two separate 5 mg films, applied in different parts of the buccal cavity of the dog, led to a significant increase in the plasma ketamine levels.

Surprisingly, the total area under the curve during the 480-minute time course experiment was higher for all of the film formulations than for the intravenous dose of ketamine (F4).

In conclusion, buccal placement of the ketamine formulations appears to provide a surprisingly high bioavailbility and total exposure when compared with intravenous formulations.

**Example 5: Comparison of physical properties of placebo alginate films as used in the present invention with alternative alginate films and pullulan films**

***Preparation of alginate and pullulan films***

Films were prepared from the following film-forming agents:

- Protanal® LFR 5/60 - a low weight sodium alginate with a mannuronate:guluronate (M:G) ratio of 25-35:65-75, and a mean molecular mass of c. 40,000 g/mol;

- Protanal® LF 120 - a high weight sodium alginate with a mannuronate:guluronate (M:G) ratio of 55-65:35-45, and a mean molecular mass of >90,000 g/mol; and

- Pullulan.

The alginate films were prepared using an analogous protocol to Example 1, by mixing 26.7 g sodium alginate, 197 g water, 7 g sorbitol and 7 g glycerol, with phosphoric acid used to buffer the solution to pH 5.0.
The pullulan film was prepared by mixing 25 g pullulan, 195 g water, 7 g sorbitol and 7 g glycerol in a mixer for 30 minutes, and then casting with a 1 mm gap with a blade onto a glass surface. The wet film is then dried in a drying cabinet for 1 h at 55°C. Two different final films were prepared, one containing 8.5% residual moisture and the other containing 4% residual moisture.

***Comparison of film properties in volunteer subjects***

The dissolution properties of the placebo film preparations were tested and evaluated by placing film pieces in the oral cavity of eight adult volunteer subjects. The subjects were asked to rate the time for the film to dissolve and the feel in the mouth. The results are set out in Table 13 below.
Protanal® LFR 5/60 was found to be the preferred film-forming agent film. This film product showed prolonged adhesiveness and was reported to be attached to the mucosa during the entire dissolution time (2-5 min).

*Table 13. Properties of alginate and pullulan films applied to the oral mucosa of volunteer subjects. Results for dissolution time presented as the mean across the eight subjects.*

| Test alginate polymer | Dissolution (min) | Adhesion | Comments |
|---|---|---|---|
| Protanal® LFR 5/60 alginate (low viscosity) | 2-5 | High | Good adhesion. Dissolves completely and matrix is adhesive until completely dissolved. |
| Protanal® LFR 120 alginate (high viscosity) | More than 30 | None | Not sticky. Becomes a slimy tablet which does not appear to dissolve. |
| Pullulan (8.5% residual moisture) | Less than 1 | Low | Gluey film with a rubber-like consistency. Adheres only briefly to the mucosa after which the matrix becomes stringy, loses its shape and spreads out in a much larger surface than alginate films. |
| Pullulan (4% residual moisture) | Significantly less than 1 | Low | Brittle film. Cannot be handled without breaking. |

***Comparison of adhesive force of film products***

The relative adhesive properties of some of the film products were characterized using standardized measurements of adhesion to glass plates as a surrogate for mucosal adhesion. The measurements were conducted at 25°C using a texture analyzer (model TA.XT plus C, provided by Stable Micro System, UK) with cylindrical probe (25 mm in diameter, model SMS P/25) using standardized wetting and adhesion assessment protocols.
The results of these measurements for placebo films formed from sodium alginate (Protanal® LFR 5/60) and pullulan (8.5% residual moisture) with identical proportions of the plasticizers sorbitol and glycerol) are given in Table 14 below. These measurements reflect the initial adhesive force during wetting of the film. Once wetted, as noted in the human placebo tests, the films formed from pullulan exhibit detachment behaviour inconsistent with continued delivery of drugs across the oral mucosa.

*Table 14. Mean Adhesion Force of various film products. Measurements are the mean (SD) of 5-10 separate measurements of adhesive force required to remove the film from a glass substrate.*

| Film-Forming Polymer | Protanal® 5/60 | Pullulan |
|---|---|---|
| Mean Adhesion Force (gm) | 2356 | 1761 |

(continued)

| Film-Forming Polymer | Protanal® 5/60 | Pullulan |
|---|---|---|
| SD | 279 | 216 |

***Comparison of dissolution properties of film products***

Controlled dissolution time is an additional factor in the effective delivery of drug molecules to the oral mucosa. The characteristics required for effective drug delivery from an oral mucosal film are not only continuous adhesion to the mucosal surface, but also a dissolution time that is consistent with the absorption rate of the drug into the mucosal surface.

The primary process governing the absorption of active ingredients from a muco-adhesive film is Fick's Law of diffusion of a solute, which (in its simplest form) is given below:

$$J = -D\frac{dc}{dx}$$

where:

$J$ = the rate of diffusion flux (atoms/area/time)
D = the diffusion coefficient of the solute ($cm/sec^2$)
$dc$ = change in concentration of the solute (in the film) ($mg/cm^2$)
$dx$ = change in thickness of the layer to be penetrated (mucosal thickness and unstirred water layer)(mm)

Thus, for a given active ingredient, presented the mucosal membrane in its 'absorbable' form by the film, the key variable in the rate and extent of absorption is *dc* - the concentration of the active ingredient in the film. A high concentration of the active ingredient(s) in the film produces a very high concentration gradient between the film and mucosal layer resulting a rapid burst of absorption while the film concentration and tissue concentration equilibrate (dictated by *D*).

In a static state, the absorption from the film would stop when the concentration in the film reaches the concentration in the underlying tissue. However, complete absorption is facilitated by the properties of the mucosal tissue by virtue of the fact that the tissue is constantly perfused by both arterial and venous blood, thereby removing absorbed active ingredient from the local area and maintaining the concentration gradient (mass-imbalance) between the film and tissue. In this regard, the tissue and its blood supply can be regarded as a 'sink' into which the active ingredient is constantly being drawn from the dosage form.

Finally, alginate polymers as a dry formulation functionally lower the penetration layer thickness due to their immediate absorption of the unstirred water layer into the alginate matrix. Removal of the unstirred water layer fundamentally differs from liquid-based oral or nasal sprays and allows the film to present both API and any membrane permeability enhancers directly to a more hydrophobic environment: mucin (if present) and the plasma membrane of the absorbing epithelium.

Thus, while muco-adhesive properties are important, it is ***the combination*** of constant adhesion to mucosal surface, removal of the unstirred water layer ***and*** controlled dissolution rate which allows a consistent and complete delivery of the drugs with diffusion coefficients in the 'normal' range. Rapid dissolution will allow an excessive proportion of the dose to be released prior to diffusion into the mucosa into the saliva and swallowed, effectively lowering the concentration gradient, *dc*, and thereby negating effective trans-mucosal delivery.

In order to address this property, the Protanal® LFR 5/60 and pullulan films were compared for their relative disintegration times using a standard disintegration test (50 mL PBS, pH 6.8 at 25°C with 50 rpm stirring bar, visual inspection of complete disintegration). The results are given in Table 15 below and show that pullulan films, under standard disintegration testing procedures, disintegrate at twice the rate of the alginate film.

*Table 15. Disintegration times of Protanal® LFR 5/60 and Pullulan-based films. Results are the mean of 5 independent experiments and are presented as mean (SD).*

| Film-Forming Polymer | Pullulan | Alginate (Protanal® 5/60) |
|---|---|---|
| Disintegration Time (sec) | 74 | 141 |

(continued)

| Film-Forming Polymer | Pullulan | Alginate (Protanal® 5/60) |
|---|---|---|
| SD | 5 | 8 |

***Conclusions***

The following conclusions can be drawn from this example.

1. In human volunteers, the alginate films adhere to the mucosa instantly and the matrix is adhesive during the whole dissolution time. The pullulan films only briefly adhere to a mucosa but the matrix is rapidly dissolved and disintegrate to non-adhesive matrix that easily becomes part of the saliva matrix. The alginate films with a lower M:G ratio and lower molecular weight (e.g. Protanal® LFR 5/60) perform better in these tests than alginate films with a higher M:G ratio and higher molecular weight.

2. The alginate films with a lower M:G ratio and lower molecular weight adhere with greater force to a surface than the alginate films with a higher M:G ratio and higher molecular weight, or the pullulan films.

3. The pullulan film dissolution times are inconsistent with effective trans-mucosal delivery, dissolving too rapidly to effectively deliver active ingredients into the oral mucosa, even if they were to remain adhered to the mucosal surface. In contrast, alginate films such as Protanal® LFR 5/60 have a controlled dissolution rate that allows for improved continual delivery of drugs across the oral mucosa.

4. The pullulan film, if dried to levels which make it physically robust (e.g. 8.5% residual moisture), assumes a rubber-like consistency that is inconsistent with a pharmaceutical product. If further drying is attempted (e.g. to 4% residual moisture), the film becomes too fragile to effectively manufacture.

The results therefore indicate that films comprised of alginate perform better as oral transmucosal films than films comprised of pullulan. Further, of the alginate-containing films, the alginates with a lower molecular weight and which comprise 25-35 wt% mannuronate and 65-75 wt% guluronate perform better than alginate films having a molecular weight >90,000 g/mol and a higher M:G ratio.

**References**

[1] Muller, J.; Pentyalam S.; Dilger, J.; Pentyalm S. Ketamine enantiomers in the rapid and sustained antidepressant effects. Ther Adv Psychopharmacol, 2016, 6, 185-192.

[2] Haas, D. A. and Harper, D. G. Ketamine: A Review of Its Pharmacologic Properties and Use in Ambulatory Anesthesia. Anesth Prog, 1992, 39, 61-68.

[3] Sinner, B.; Graf, B.M. "Ketamine" (2008). In "Modern Anesthetics. Handbook of Experimental Pharmacology", Schüttler, J.; Schwilden, H. (eds.) 182, 313-33.

[4] Prachayasittikul, V.; Isarankura-Na-Ayudhya, C.; Tantimongcolwat, T.; Nantasenamat, C.; Galla, H.J. EDTA-induced Membrane Fluidization and Destabilization: Biophysical Studies on Artificial Lipid Membranes. Acta biochimica et biophysica Sinica, 2007, 39(11), 901-913.

[5] Managaro, A.; Wertz, P. The effect of permeabilizer on the in vitro penetration of propranolol through porcine buccal epithelium.

[6] Date, A.A.; Desai, N.; Dixit, R.; Nagarsenker, M. Self-nanoemulsifying Drug Delivery Systems: Formulation Insights, Applications and Advances. Nanomedicine, 2010, 5(10), 1595-1616.

[7] Pouton, C.W. Formation of poorly water-soluble drugs for oral administration: Physicochemical and physiological issues and the lipid formulation classification system. European Journal of Pharmaceutical Sciences, 2006, 29(3-4),

278-287.

[8] PCT/SE2006/050626

[9] European Pharmacopoeia 8, 2565.

[10] Marttin, E. et al. The effect of methylated b-cyclodextrins on the tight junctions of the rat nasal respiratory epithelium: Electron microscopic and confocal laser scanning microscopic visualization studies. J Control Release, 1999, 57, 205-213.

[11] Merkus, F. W. H. M. et al. Cyclodextrins in nasal drug delivery. Adv Drug Del Rev 1999, 36, 41-57.

[12] Shannon, R. D. Revised effective ionic radii and systematic studies of interatomic distances in halides and chalcogenides. Acta Cryst. A, 1976, 32(5), 751-767.

## Claims

**1.** A film suitable for administration to an oral cavity comprising:

(i) an alginate salt of a monovalent cation or a mixture of alginate salts containing at least one alginate salt of a monovalent cation;
(ii) an active pharmaceutical ingredient (API) which is a compound of Formula (I)

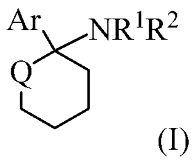

(I)

wherein:

Ar is selected from

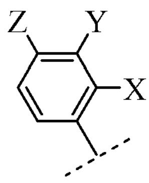 and 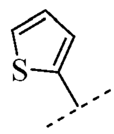 ;

X is selected from hydrogen, halo, OH, $NH_2$, methyl, trifluoromethyl and methoxy;
Y is selected from hydrogen, halo, OH, $NH_2$, methyl, trifluoromethyl and methoxy;
Z is selected from hydrogen, halo, OH, $NH_2$, methyl, trifluoromethyl and methoxy;
Q is selected from $-CH_2-$, -CH(OH)-, -CH(Me)-, -CH(OMe)-, -(C=O)-, -(C=S)- and -(C=NR)-, wherein R is selected from hydrogen or $C_{1-6}$ alkyl;
$R^1$ is selected from hydrogen and $C_{1-6}$ alkyl and $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl, or $R^1$ and $R^2$ are linked so as to form a bivalent alkylene moiety having from 3 to 7 carbon atoms; and

(iii) an acid $H_xA$, wherein A is a counterion having an ionic radius of 2.65 Å or greater, and x is a positive integer which is equal to the charge on the counterion A;

further wherein the alginate salt of a monovalent cation (a) comprises from 25 to 35% by weight of β-D-mannuronate and/or from 65 to 75% by weight of α-L-guluronate, and (b) has a weight average molecular weight of from 30,000 g/mol to 90,000 g/mol.

**2.** The film according to claim 1, wherein the van der Waals volume of the counterion A is 45 $Å^3$ or greater.

**3.** The film according to claim 1 or claim 2, wherein the acid $H_xA$ is selected from acetic acid, ascorbic acid, phosphoric acid, citric acid, tartaric acid, acrylic acid, poly(acrylic) acid, iodic acid, malic acid, methanesulfonic acid and combinations thereof, and preferably wherein the acid $H_xA$ is phosphoric acid.

**4.** The film according to any one of claims 1 to 3, wherein the film further comprises an additive selected from xylitol, a cyclodextrin, poly(vinyl pyrrolidone), hydroxypropylmethylcellulose, poly(acrylic acid) and pullulan.

**5.** A film suitable for administration to an oral cavity comprising:

(i) an alginate salt of a monovalent cation or a mixture of alginate salts containing at least one alginate salt of a monovalent cation;
(ii) an active pharmaceutical ingredient (API) which is a pharmaceutically acceptable salt of a compound of Formula (I)

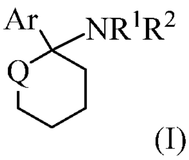

(I)

wherein:

Ar is selected from

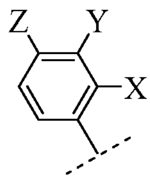 and 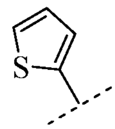 ;

X is selected from hydrogen, halo, OH, $NH_2$, methyl, trifluoromethyl and methoxy;
Y is selected from hydrogen, halo, OH, $NH_2$, methyl, trifluoromethyl and methoxy;
Z is selected from hydrogen, halo, OH, $NH_2$, methyl, trifluoromethyl and methoxy;
Q is selected from $-CH_2-$, -CH(OH)-, -CH(Me)-, -CH(OMe)-, -(C=O)-, -(C=S)- and -(C=NR)-, wherein R is selected from hydrogen or $C_{1-6}$ alkyl;
$R^1$ is selected from hydrogen and $C_{1-6}$ alkyl and $R^2$ is selected from hydrogen, $C_{1-6}$ alkyl optionally substituted with halo, hydroxyl, $C_{1-4}$ alkoxy, amino or $C_{1-4}$ alkylamino, and $C_{1-6}$ alkenyl, or $R^1$ and $R^2$ are linked so as to form a bivalent alkylene moiety having from 3 to 7 carbon atoms; and

(iii) an additive selected from xylitol, a cyclodextrin, poly(vinyl pyrrolidone), hydroxypropylmethylcellulose, poly(acrylic acid) and pullulan;

further wherein the alginate salt of a monovalent cation (a) comprises from 25 to 35% by weight of β-D-mannuronate and/or from 65 to 75% by weight of α-L-guluronate, and (b) has a weight average molecular weight of from 30,000 g/mol to 90,000 g/mol.

**6.** The film according to claim 5, wherein additive (iii) is poly(acrylic acid).

**7.** The film according to claim 5 or claim 6, wherein:

(a) the weight/weight ratio of the pharmaceutically acceptable salt of the compound of Formula (I) to additive (iii) is from 1:1 to 1:20; and/or
(b) the film further comprises an acid $H_xA$, wherein A is a counterion having an ionic radius of 2.65 Å or greater,

and x is a positive integer which is equal to the charge on the counterion A; and/or
(c) the pharmaceutically acceptable salt is a hydrochloride salt.

**8.** The film according to any one of claims 1 to 7, wherein in Formula (I):

X is selected from hydrogen or halo;
Y is selected from hydrogen or methoxy;
Z is hydrogen;
Q is selected from $-CH_2-$ or -CO-;
$R^1$ is hydrogen and $R^2$ is selected from methyl or ethyl, or $R^1$ and $R^2$ together form hexylene.

**9.** The film according to any one of claims 1 to 8, wherein:

(a) the API is selected from ketamine, tiletamine or a pharmaceutically acceptable salt thereof, preferably wherein the API is ketamine, more preferably racemic ketamine, (R)-ketamine or (S)-ketamine, or a pharmaceutically acceptable salt thereof; and/or
(b) the alginate salt of a monovalent cation is selected from a sodium alginate, a potassium alginate and an ammonium alginate, preferably a sodium alginate; and/or
(c) the film comprises from 25% to 99% by weight of the alginate salt of a monovalent cation or the mixture of alginate salts containing at least one alginate salt of a monovalent cation, from 0% to 20% by weight of water, and from 0.001% to 75% by weight of the API, preferably from 29% to 93% by weight of the alginate salt of a monovalent cation or the mixture of alginate salts containing at least one alginate salt of a monovalent cation, from 5% to 15% by weight of water, and from 0.15% to 50% by weight of the API; and/or
(d) the film further comprises:

at least one plasticizer which is selected from sorbitol, glycerol, and a combination thereof, preferably both sorbitol and glycerol; and
optionally, a basifying agent which is optionally aqueous sodium hydroxide; preferably wherein the film further comprises from 0% to 40% by weight of sorbitol, and from 0% to 40% by weight of glycerol.

**10.** A film according to any one of claims 1 to 9 for use in the treatment of a human patient.

**11.** A film according to any one of claims 1 to 9 for use in anesthesia, pain management, or the treatment of a condition selected from amnesia, depression and bipolar disorder, in a human patient.

**12.** A film for use according to claim 10 or claim 11, wherein the film is administered to the oral cavity of the human patient.

**13.** A method of manufacturing a film according to any one of claims 1 to 4, 8 or 9, said method comprising the following steps:

(a) mixing the API in water, and optionally subsequently adjusting the pH of the solution to the desired level by addition of an appropriate acid or base, typically a concentrated acid, and preferably adjusting the pH of the solution to from 2 to 4;
(b) optionally, mixing one or more excipients into the solution;
(c) adding the alginate salt of monovalent cation under suitable conditions to result in the formation of a viscous cast;
(d) adjusting the pH of the solution to the desired level by addition of an appropriate acid or base, typically a diluted acid or alkali, preferably a diluted alkali, and preferably adjusting the pH of the solution to from 3 to 5;
(e) optionally, sonicating the cast;
(f) leaving the cast to de-aerate;
(g) pouring the cast onto a surface and spreading the cast out to the desired thickness;
(h) drying the cast layer, typically at a temperature of from 30 to 70 °C until the residual water content of the film is from 0 to 20% by weight and a solid film is formed; and
(i) optionally, cutting the solid film into pieces of the desired size, further optionally placing these pieces into pouches, preferably wherein the pouches are made from PET-lined aluminium, sealing the pouches and further optionally, labelling them.

**14.** A method of manufacturing a film according to any one of claims 5 to 9, said method comprising the following steps:

(a) mixing the salt of the API in water;
(b) adding one or more additives selected from xylitol, a cyclodextrin, poly(vinyl pyrrolidone), hydroxypropylmethylcellulose, poly(acrylic acid) and pullulan to the solution;
(c) optionally, mixing one or more excipients into the solution;
(d) adding the alginate salt of monovalent cation under suitable conditions to result in the formation of a viscous cast;
(e) optionally, adding further water to the cast;
(f) optionally, sonicating the cast;
(g) leaving the cast to de-aerate;
(h) pouring the cast onto a surface and spreading the cast out to the desired thickness;
(i) drying the cast layer, typically at a temperature of from 30 to 70 °C until the residual water content of the film is from 0 to 20% by weight and a solid film is formed; and
(j) optionally, cutting the solid film into pieces of the desired size, further optionally placing these pieces into pouches, preferably wherein the pouches are made from PET-lined aluminium, sealing the pouches and further optionally, labelling them.

**15.** The method of claim 13 or claim 14, wherein after the viscous cast is poured onto a surface, it is first spread out to a thickness of about 2 mm by means of an applicator with a slit height of about 2 mm, and is then subsequently spread out to a thickness of about 1 mm by means of an applicator with a slit height of about 1 mm.

**Patentansprüche**

**1.** Film, der zur Verabreichung in einer Mundhöhle geeignet ist und Folgendes umfasst:

(i) ein Alginatsalz eines einwertigen Kations oder ein Gemisch von Alginatsalzen, das mindestens ein Alginatsalz eines einwertigen Kations enthält;
(ii) einen pharmazeutischen Wirkstoff (Active Pharmaceutical Ingredient, API), der eine Verbindung der Formel (I) ist:

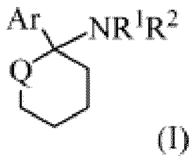

(I)

wobei:

Ar ausgewählt ist aus

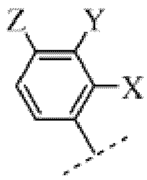

und

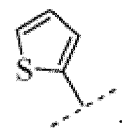

;

X ausgewählt ist aus Wasserstoff, Halogen, OH, $NH_2$, Methyl, Trifluormethyl und Methoxy;
Y ausgewählt ist aus Wasserstoff, Halogen, OH, $NH_2$, Methyl, Trifluormethyl und Methoxy;
Z ausgewählt ist aus Wasserstoff, Halogen, OH, $NH_2$, Methyl, Trifluormethyl und Methoxy;
Q ausgewählt ist aus $-CH_2-$, -CH(OH)-, -CH(Me)-, -CH(OMe)-, - (C=O)-, -(C=S)- und -(C=NR)-, wobei R ausgewählt ist aus Wasserstoff oder $C_{1-6}$-Alkyl;
$R^1$ ausgewählt ist aus Wasserstoff und $C_{1-6}$-Alkyl und $R^2$ ausgewählt ist aus Wasserstoff, $C_{1-6}$-Alkyl, optional substituiert mit Halogen, Hydroxyl, $C_{1-4}$-Alkoxy, Amino oder $C_{1-4}$-Alkylamino, und $C_{1-6}$-Alkenyl, oder $R^1$ und $R^2$ so verbunden sind, dass sie eine zweiwertige Alkyleneinheit mit 3 bis 7 Kohlenstoffatomen bilden; und

(iii) eine Säure $H_xA$, wobei A ein Gegenion mit einem Ionenradius von 2,65 Å oder größer ist und x eine positive ganze Zahl ist, die gleich der Ladung des Gegenions A ist;

wobei ferner das Alginatsalz eines einwertigen Kations (a) 25 bis 35 Gew.-% β-D-Mannuronat und/oder 65 bis 75 Gew.-% α-L-Guluronat umfasst und (b) ein gewichtsmittleres Molekulargewicht von 30.000 g/mol bis 90.000 g/mol aufweist.

**2.** Film nach Anspruch 1, wobei das Van-der-Waals-Volumen des Gegenions A 45 $Å^3$ oder größer ist.

**3.** Film nach Anspruch 1 oder 2, wobei die Säure $H_xA$ aus Essigsäure, Ascorbinsäure, Phosphorsäure, Zitronensäure, Weinsäure, Acrylsäure, Polyacrylsäure, Iodsäure, Äpfelsäure, Methansulfonsäure und Kombinationen davon ausgewählt ist, und wobei die Säure $H_xA$ vorzugsweise Phosphorsäure ist.

**4.** Film nach einem der Ansprüche 1 bis 3, wobei der Film ferner ein Additiv umfasst, das aus Xylit, einem Cyclodextrin, Poly(vinylpyrrolidon), Hydroxypropylmethylcellulose, Poly(acrylsäure) und Pullulan ausgewählt ist.

**5.** Film, der zur Verabreichung in einer Mundhöhle geeignet ist und Folgendes umfasst:

(i) ein Alginatsalz eines einwertigen Kations oder ein Gemisch von Alginatsalzen, das mindestens ein Alginatsalz eines einwertigen Kations enthält;
(ii) einen pharmazeutischen Wirkstoff (API), der ein pharmazeutisch annehmbares Salz einer Verbindung der Formel (I) ist:

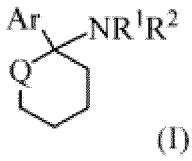

(I)

wobei:

Ar ausgewählt ist aus

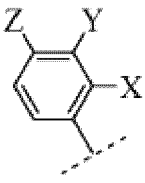

und

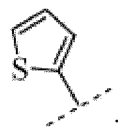

;

X ausgewählt ist aus Wasserstoff, Halogen, OH, $NH_2$, Methyl, Trifluormethyl und Methoxy;
Y ausgewählt ist aus Wasserstoff, Halogen, OH, $NH_2$, Methyl, Trifluormethyl und Methoxy;
Z ausgewählt ist aus Wasserstoff, Halogen, OH, $NH_2$, Methyl, Trifluormethyl und Methoxy;
Q ausgewählt ist aus $-CH_2-$, -CH(OH)-, -CH(Me)-, -CH(OMe)-, - (C=O)-, -(C=S)- und -(C=NR)-, wobei R ausgewählt ist aus Wasserstoff oder $C_{1-6}$-Alkyl;
$R^1$ ausgewählt ist aus Wasserstoff und $C_{1-6}$-Alkyl und $R^2$ ausgewählt ist aus Wasserstoff, $C_{1-6}$-Alkyl, optional substituiert mit Halogen, Hydroxyl, $C_{1-4}$-Alkoxy, Amino oder $C_{1-4}$-Alkylamino, und $C_{1-6}$-Alkenyl, oder $R^1$ und $R^2$ so verbunden sind, dass sie eine zweiwertige Alkyleneinheit mit 3 bis 7 Kohlenstoffatomen bilden; und

(iii) ein Additiv, ausgewählt aus Xylitol, einem Cyclodextrin, Poly(vinylpyrrolidon), Hydroxypropylmethylcellulose, Poly(acrylsäure) und Pullulan;

wobei ferner das Alginatsalz eines einwertigen Kations (a) 25 bis 35 Gew.-% β-D-Mannuronat und/oder 65 bis 75 Gew.-% α-L-Guluronat umfasst und (b) ein gewichtsmittleres Molekulargewicht von 30.000 g/mol bis 90.000 g/mol aufweist.

**6.** Film nach Anspruch 5, wobei das Additiv (iii) Poly(acrylsäure) ist.

**7.** Film nach Anspruch 5 oder 6, wobei:

(a) das Gewichtsverhältnis des pharmazeutisch annehmbaren Salzes der Verbindung der Formel (I) zu Additiv (iii) 1:1 bis 1:20 beträgt; und/oder
(b) der Film ferner eine Säure $H_xA$ umfasst, wobei A ein Gegenion mit einem Ionenradius von 2,65 Å oder größer ist und x eine positive ganze Zahl ist, die gleich der Ladung des Gegenions A ist; und/oder
(c) das pharmazeutisch annehmbare Salz ein Hydrochloridsalz ist.

**8.** Film nach einem der Ansprüche 1 bis 7, wobei in Formel (I):

X aus Wasserstoff oder Halogen ausgewählt ist;
Y aus Wasserstoff oder Methoxy ausgewählt ist;
Z Wasserstoff ist;
Q aus $-CH_2-$ oder -CO- ausgewählt ist;
$R^1$ Wasserstoff ist und $R^2$ aus Methyl oder Ethyl ausgewählt ist oder $R^1$ und $R^2$ zusammen Hexylen bilden.

**9.** Film nach einem der Ansprüche 1 bis 8, wobei:

(a) der Wirkstoff ausgewählt ist aus Ketamin, Tiletamin oder einem pharmazeutisch annehmbaren Salz davon, vorzugsweise wobei der Wirkstoff Ketamin ist, stärker bevorzugt racemisches Ketamin, (R)-Ketamin oder (S)-Ketamin, oder ein pharmazeutisch annehmbares Salz davon; und/oder
(b) das Alginatsalz eines einwertigen Kations aus einem Natriumalginat, einem Kaliumalginat und einem Ammoniumalginat, vorzugsweise einem Natriumalginat, ausgewählt ist; und/oder
(c) der Film 25 bis 99 Gew.-% des Alginatsalzes eines einwertigen Kations oder des Gemischs von Alginatsalzen umfasst; das mindestens ein Alginatsalz eines einwertigen Kations enthält, 0 bis 20 Gew.-% Wasser und 0,001 bis 75 Gew.-% des API, vorzugsweise 29 bis 93 Gew.-% des Alginatsalzes eines einwertigen Kations oder des Gemischs von Alginatsalzen das mindestens ein Alginatsalz eines einwertigen Kations enthält, 5 bis 15 Gew.-% Wasser und 0,15 bis 50 Gew.-% des API; und/oder
(d) der Film ferner umfasst:

mindestens einen Weichmacher, der aus Sorbit, Glycerin und einer Kombination davon, vorzugsweise sowohl Sorbit als auch Glycerin, ausgewählt ist; und
optional ein Basenbildner, der optional wässriges Natriumhydroxid ist; vorzugsweise wobei der Film ferner 0 bis 40 Gew.-% Sorbit und 0 bis 40 Gew.-% Glycerin umfasst.

**10.** Film nach einem der Ansprüche 1 bis 9 zur Verwendung bei der Behandlung eines menschlichen Patienten.

**11.** Film nach einem der Ansprüche 1 bis 9 zur Verwendung bei der Anästhesie, der Schmerzbehandlung oder der Behandlung eines Zustands, ausgewählt aus Amnesie, Depression und bipolarer Störung, bei einem menschlichen Patienten.

**12.** Film zur Verwendung nach Anspruch 10 oder Anspruch 11, wobei der Film in die Mundhöhle des menschlichen Patienten verabreicht wird.

**13.** Verfahren zur Herstellung eines Films nach einem der Ansprüche 1 bis 4, 8 oder 9, wobei das Verfahren folgende Schritte umfasst:

(a) Mischen des API in Wasser und optional anschließendes Einstellen des pH-Werts der Lösung auf den gewünschten Wert durch Zugabe einer geeigneten Säure oder Base, typischerweise einer konzentrierten Säure, und vorzugsweise Einstellen des pH-Werts der Lösung auf 2 bis 4;
(b) optional Mischen eines oder mehrerer Hilfsstoffe in die Lösung;
(c) Zugeben des Alginatsalzes eines einwertigen Kations unter geeigneten Bedingungen, um die Bildung eines viskosen Gusses zu bewirken;
(d) Einstellen des pH-Werts der Lösung auf den gewünschten Wert durch Zugeben einer geeigneten Säure oder Base, typischerweise einer verdünnten Säure oder Lauge, vorzugsweise einer verdünnten Lauge, und vorzugsweise Einstellen des pH-Werts der Lösung auf 3 bis 5;

(e) optional Ultraschallbehandeln des Gusses;
(f) Entlüftenlassen des Gusses;
(g) Gießen des Gusses auf eine Oberfläche und Ausbreiten des Gusses auf die gewünschte Dicke;
(h) Trocknen der Gussschicht, typischerweise bei einer Temperatur von 30 bis 70 °C, bis der Restwassergehalt des Films 0 bis 20 Gew.-% beträgt und ein fester Film gebildet wird; und
(i) optional Schneiden des festen Films in Stücke der gewünschten Größe, optional weiteres Einbringen dieser Stücke in Beutel, wobei die Beutel vorzugsweise aus mit PET ausgekleidetem Aluminium bestehen, Versiegeln der Beutel und optional weiteres Etikettieren derselben.

**14.** Verfahren zur Herstellung eines Films nach einem der Ansprüche 5 bis 9, wobei das Verfahren folgende Schritte umfasst:

(a) Mischen des Salzes des API in Wasser;
(b) Zugeben eines oder mehrerer Additive, ausgewählt aus Xylitol, einem Cyclodextrin, Poly(vinylpyrrolidon), Hydroxypropylmethylcellulose, Poly(acrylsäure) und Pullulan, zu der Lösung;
(c) optional Mischen eines oder mehrerer Hilfsstoffe in die Lösung;
(d) Zugeben des Alginatsalzes eines einwertigen Kations unter geeigneten Bedingungen, um die Bildung eines viskosen Gusses zu bewirken;
(e) optional Zugeben von weiterem Wasser zu dem Guss;
(f) optional Beschallen des Gusses;
(g) Entlüftenlassen des Gusses;
(h) Gießen des Gusses auf eine Oberfläche und Ausbreiten des Gusses auf die gewünschte Dicke;
(i) Trocknen der Gussschicht, typischerweise bei einer Temperatur von 30 bis 70 °C, bis der Restwassergehalt des Films 0 bis 20 Gew.-% beträgt und ein fester Film gebildet wird; und
(j) optional Schneiden des festen Films in Stücke der gewünschten Größe, optional weiteres Einbringen dieser Stücke in Beutel, wobei die Beutel vorzugsweise aus mit PET ausgekleidetem Aluminium bestehen, Versiegeln der Beutel und optional weiteres Etikettieren derselben.

**15.** Verfahren nach Anspruch 13 oder Anspruch 14, wobei der viskose Guss, nachdem er auf eine Oberfläche gegossen wurde, zunächst mit Hilfe eines Applikators mit einer Schlitzhöhe von etwa 2 mm auf eine Dicke von etwa 2 mm ausgebreitet wird, und anschließend mit Hilfe eines Applikators mit einer Schlitzhöhe von etwa 1 mm auf eine Dicke von etwa 1 mm ausgebreitet wird.

## Revendications

**1.** Film apte à une administration dans la cavité buccale comprenant :

(i) un sel d'alginate d'un cation monovalent ou un mélange de sels d'alginate contenant au moins un sel d'alginate d'un cation monovalent ;
(ii) un principe actif (PA) consistant en un composé de formule (I)

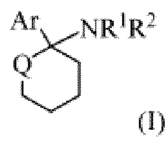
(I)

dans laquelle :

Ar est choisi parmi

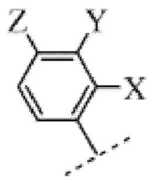 et 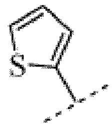 ;

X est choisi parmi l'hydrogène, un groupe halogéno, OH, $NH_2$, méthyle, trifluorométhyle et méthoxy ;
Y est choisi parmi l'hydrogène, un groupe halogéno, OH, $NH_2$, méthyle, trifluorométhyle et méthoxy ;
Z est choisi parmi l'hydrogène, un groupe halogéno, OH, $NH_2$, méthyle, trifluorométhyle et méthoxy ;
Q est choisi parmi $-CH_2-$, -CH(OH)-, -CH(Me)-, -CH(OMe)-, -(C=O)-, - (C=S)- et -(C=NR)-, où R est choisi parmi l' hydrogène et un alkyle en $C_{1-6}$ ;
$R^1$ est choisi parmi l'hydrogène et un alkyle en $C_{1-6}$ et $R^2$ est choisi parmi l'hydrogène, un alkyle en $C_{1-6}$ éventuellement substitué par halogéno, hydroxyle, alcoxy en $C_{1-4}$, amino, ou alkylamino en $C_{1-4}$, et un alcényle en $C_{1-6}$, ou bien $R^1$ et $R^2$ sont liés de manière à former un fragment alkylène bivalent ayant de 3 à 7 atomes de carbone ; et

(iii) un acide $H_xA$, dans lequel A est un contre-ion ayant un rayon ionique de 2,65 Å ou plus, et x est un entier positif égal à la charge du contre-ion A ; et de surcroît le sel d'alginate d'un cation monovalent (a) comprend 25 à 35 % en poids de β-D-mannuronate et/ou 65 à 75 % en poids d'α-L-guluronate, et (b) a un poids moléculaire moyen en poids de 30 000 g/mol à 90 000 g/mol.

**2.** Film selon la revendication 1, dans lequel le volume de van der Waals du contre-ion A est de 45 $Å^3$ ou plus.

**3.** Film selon la revendication 1 ou la revendication 2, dans lequel l'acide $H_xA$ est choisi parmi l'acide acétique, l'acide ascorbique, l'acide phosphorique, l'acide citrique, l'acide tartrique, l'acide acrylique, l'acide polyacrylique, l'acide iodique, l'acide malique, l'acide méthanesulfonique et leurs combinaisons ; l'acide $H_xA$ étant de préférence l'acide phosphorique.

**4.** Film selon l'une quelconque des revendications 1 à 3, le film comprenant en outre un additif choisi parmi le xylitol, une cyclodextrine, la poly(vinylpyrrolidone), l'hydroxypropylméthylcellulose, le polyacide acrylique et le pullulane.

**5.** Film apte à une administration dans la cavité buccale comprenant :

(i) un sel d'alginate d'un cation monovalent ou un mélange de sels d'alginate contenant au moins un sel d'alginate d'un cation monovalent ;
(ii) un principe actif (PA) consistant en un sel pharmaceutiquement acceptable d'un composé de formule (I)

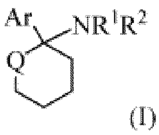
(I)

dans laquelle :

Ar est choisi parmi

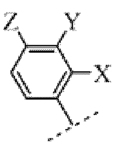 et 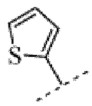 ;

X est choisi parmi l'hydrogène, un groupe halogéno, OH, $NH_2$, méthyle, trifluorométhyle et méthoxy ;
Y est choisi parmi l'hydrogène, un groupe halogéno, OH, $NH_2$, méthyle, trifluorométhyle et méthoxy ;

Z est choisi parmi l'hydrogène, un groupe halogéno, OH, $NH_2$, méthyle, trifluorométhyle et méthoxy ;
Q est choisi parmi $-CH_2-$, -CH(OH)-, -CH(Me)-, -CH(OMe)-, -(C=O)-, - (C=S)- et -(C=NR)-, où R est choisi parmi l' hydrogène et un alkyle en $C_{1-6}$ ;
$R^1$ est choisi parmi l'hydrogène et un alkyle en $C_{1-6}$ et $R^2$ est choisi parmi l'hydrogène, un alkyle en $C_{1-6}$ éventuellement substitué par halogéno, hydroxyle, alcoxy en $C_{1-4}$, amino, ou alkylamino en $C_{1-4}$, et un alcényle en $C_{1-6}$, ou bien $R^1$ et $R^2$ sont liés de manière à former un fragment alkylène bivalent ayant de 3 à 7 atomes de carbone ; et

(iii) un additif choisi parmi le xylitol, une cyclodextrine, la poly(vinylpyrrolidone), l'hydroxypropylméthylcellulose, le polyacide acrylique et le pullulane ;

et de surcroît le sel d'alginate d'un cation monovalent (a) comprend 25 à 35 % en poids de β-D-mannuronate et/ou 65 à 75 % en poids d'α-L-guluronate, et (b) a un poids moléculaire moyen en poids de 30 000 g/mol à 90 000 g/mol.

**6.** Film selon la revendication 5, dans lequel l'additif (iii) est le polyacide acrylique.

**7.** Film selon la revendication 5 ou la revendication 6, dans lequel :

(a) le rapport poids/poids du sel pharmaceutiquement acceptable du composé de formule (I) à l'additif (iii) est de 1:1 à 1:20 ; et/ou
(b) le film comprend en outre un acide $H_xA$, dans lequel A est un contre-ion ayant un rayon ionique de 2,65 Å ou plus, et x est un entier positif égal à la charge du contre-ion A; et/ou
(c) le sel pharmaceutiquement acceptable est un sel de chlorhydrate.

**8.** Film selon l'une quelconque des revendications 1 à 7, dans lequel, dans la formule (I) :

X est choisi parmi l' hydrogène et un groupe halogéno ;
Y est choisi parmi l' hydrogène et un groupe méthoxy ;
Z est l'hydrogène ;
Q est choisi parmi $-CH_2-$ ou -CO- ;
$R^1$ est l'hydrogène et $R^2$ est choisi parmi un groupe méthyle ou éthyle, ou bien $R^1$ et $R^2$ forment ensemble un groupe hexylène.

**9.** Film selon l'une quelconque des revendications 1 à 8, dans lequel :

(a) le PA est choisi parmi la kétamine, la tilétamine ou un sel pharmaceutiquement acceptable de celles-ci, le PA étant de préférence la kétamine, plus préférablement la kétamine racémique, la (R)-kétamine ou la (S)-kétamine, ou un sel pharmaceutiquement acceptable de celles-ci ; et/ou
(b) le sel d'alginate d'un cation monovalent est choisi parmi un alginate de sodium, un alginate de potassium et un alginate d'ammonium, de préférence un alginate de sodium ; et/ou
(c) le film comprend 25 à 99 % en poids du sel d'alginate d'un cation monovalent ou du mélange de sels d'alginate contenant au moins un sel d'alginate d'un cation monovalent, 0 à 20 % en poids d'eau et 0,001 à 75 % en poids du PA ; de préférence 29 à 93 % en poids du sel d'alginate d'un cation monovalent ou du mélange de sels d'alginate contenant au moins un sel d'alginate d'un cation monovalent, 5 à 15 % en poids d'eau et 0,15 à 50 % en poids du PA ; et/ou
(d) le film comprend en outre :

au moins un plastifiant qui est choisi parmi le sorbitol, le glycérol et une combinaison de ceux-ci, de préférence à la fois le sorbitol et le glycérol, et
éventuellement, un agent alcalinisant consistant éventuellement en l'hydroxyde de sodium aqueux ;

le film comprenant de préférence en outre 0 à 40 % en poids de sorbitol, et 0 à 40 % en poids de glycérol.

**10.** Film selon l'une quelconque des revendications 1 à 9 destiné à être utilisé dans le traitement d'un patient humain.

**11.** Film selon l'une quelconque des revendications 1 à 9 destiné à être utilisé dans l'anesthésie, la gestion de la douleur ou le traitement d'une affection choisie parmi l'amnésie, la dépression et le trouble bipolaire, chez un patient humain.

**12.** Film destiné à être utilisé selon la revendication 10 ou la revendication 11, dans lequel le film est administré dans la cavité buccale du patient humain.

**13.** Procédé de fabrication d'un film selon l'une quelconque des revendications 1 à 4, 8 ou 9, ledit procédé comprenant les étapes suivantes consistant à :

(a) mélanger le PA dans de l'eau, et éventuellement ajuster ultérieurement le pH de la solution au niveau souhaité par ajout d'un acide ou d'une base appropriés, généralement un acide concentré, et de préférence ajuster la solution à un pH allant de 2 à 4 ;
(b) mélanger éventuellement un ou plusieurs excipients à la solution ;
(c) ajouter le sel d'alginate de cation monovalent dans des conditions appropriées pour entraîner la formation d'une matière coulable visqueuse ;
(d) ajuster le pH de la solution au niveau souhaité par addition d'un acide ou d'une base appropriés, généralement un acide ou un alcali dilués, de préférence un alcali dilué, et de préférence ajuster la solution à un pH allant de 3 à 5 ;
(e) soumettre éventuellement la matière coulable à un traitement aux ultrasons ;
(f) laisser la matière coulable se désaérer ;
(g) verser la matière coulable sur une surface et l'étaler jusqu'à l'épaisseur souhaitée ;
(h) sécher la couche coulée, généralement à une température de 30 à 70 °C jusqu'à ce que la teneur en eau résiduelle du film soit de 0 à 20 % en poids et qu'un film solide soit formé ; et
(i) découper éventuellement le film solide en pièces de la taille souhaitée, placer éventuellement ces pièces dans des sachets, de préférence en aluminium doublé de PET, sceller les sachets et éventuellement, en outre, les étiqueter.

**14.** Procédé de fabrication d'un film selon l'une quelconque des revendications 5 à 9, ledit procédé comprenant les étapes suivantes consistant à :

(a) mélanger le sel du PA dans de l'eau ;
(b) ajouter un ou plusieurs additifs, choisis parmi le xylitol, une cyclodextrine, la poly(vinylpyrrolidone), l'hydroxypropylméthylcellulose, le polyacide acrylique et le pullulane, à la solution ;
(c) mélanger éventuellement un ou plusieurs excipients à la solution ;
(d) ajouter le sel d'alginate de cation monovalent dans des conditions appropriées pour entraîner la formation d'une matière coulable visqueuse ;
(e) ajouter éventuellement davantage d'eau à la matière coulable ;
(f) soumettre éventuellement la matière coulable à un traitement aux ultrasons ;
(g) laisser la matière coulable se désaérer ;
(h) verser la matière coulable sur une surface et l'étaler jusqu'à l'épaisseur souhaitée ;
(i) sécher la couche coulée, généralement à une température de 30 à 70 °C jusqu'à ce que la teneur en eau résiduelle du film soit de 0 à 20 % en poids et qu'un film solide soit formé ; et
(j) découper éventuellement le film solide en pièces de la taille souhaitée, placer éventuellement ces pièces dans des sachets, de préférence en aluminium doublé de PET, sceller les sachets et éventuellement, en outre, les étiqueter.

**15.** Procédé selon la revendication 13 ou la revendication 14, dans lequel, après que la matière coulable visqueuse a été versée sur une surface, elle est d'abord étalée sur une épaisseur d'environ 2 mm au moyen d'un applicateur d'une hauteur de fente d'environ 2 mm, puis sur une épaisseur d'environ 1 mm au moyen d'un applicateur d'une hauteur de fente d'environ 1 mm.

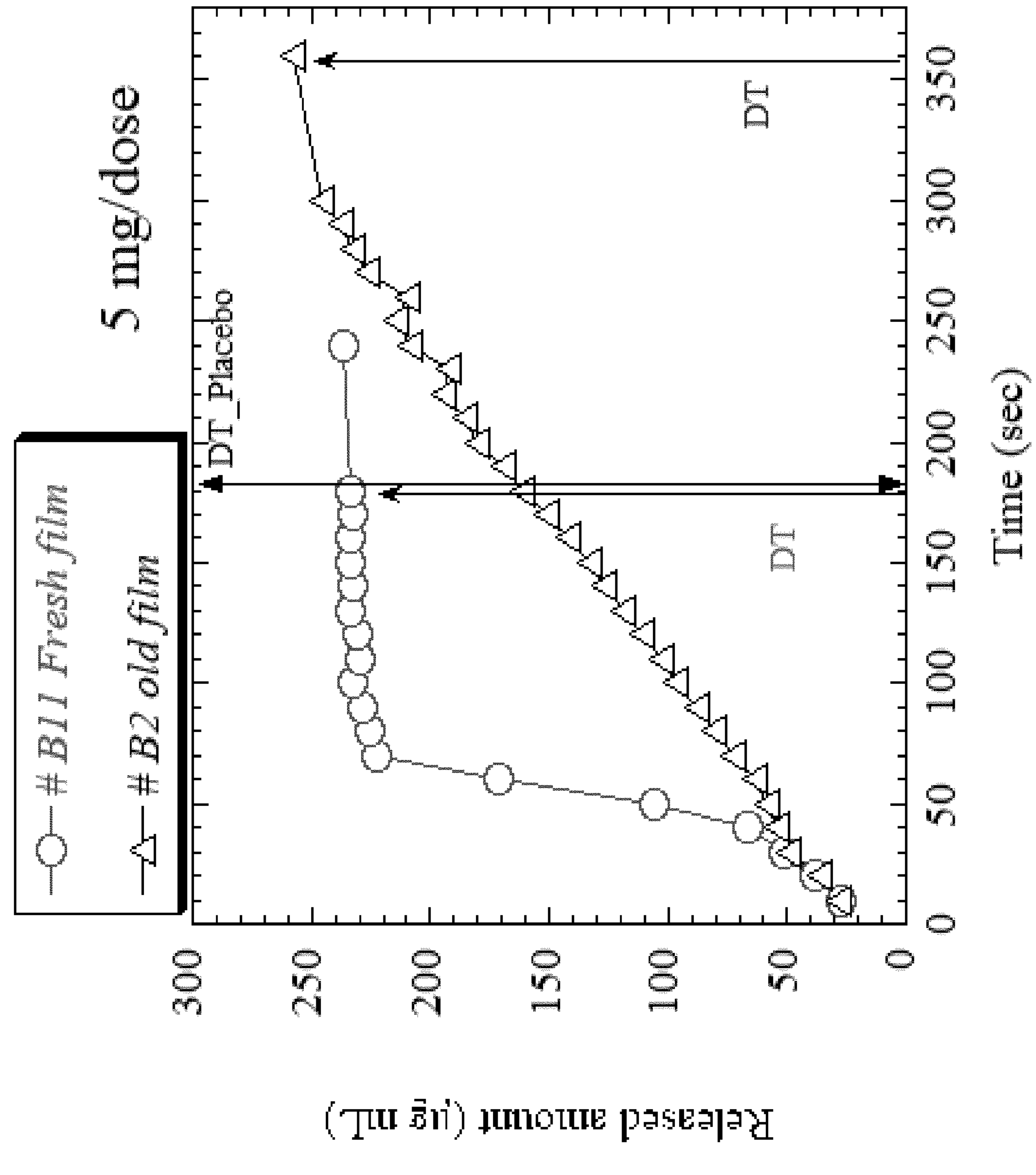

5 mg/dose
—○— # B11 Fresh film
—△— # B2 old film
DT_Placebo
DT
DT
Time (sec)
Released amount (µg mL)
0
50
100
150
200
250
300
350


Fig. 1

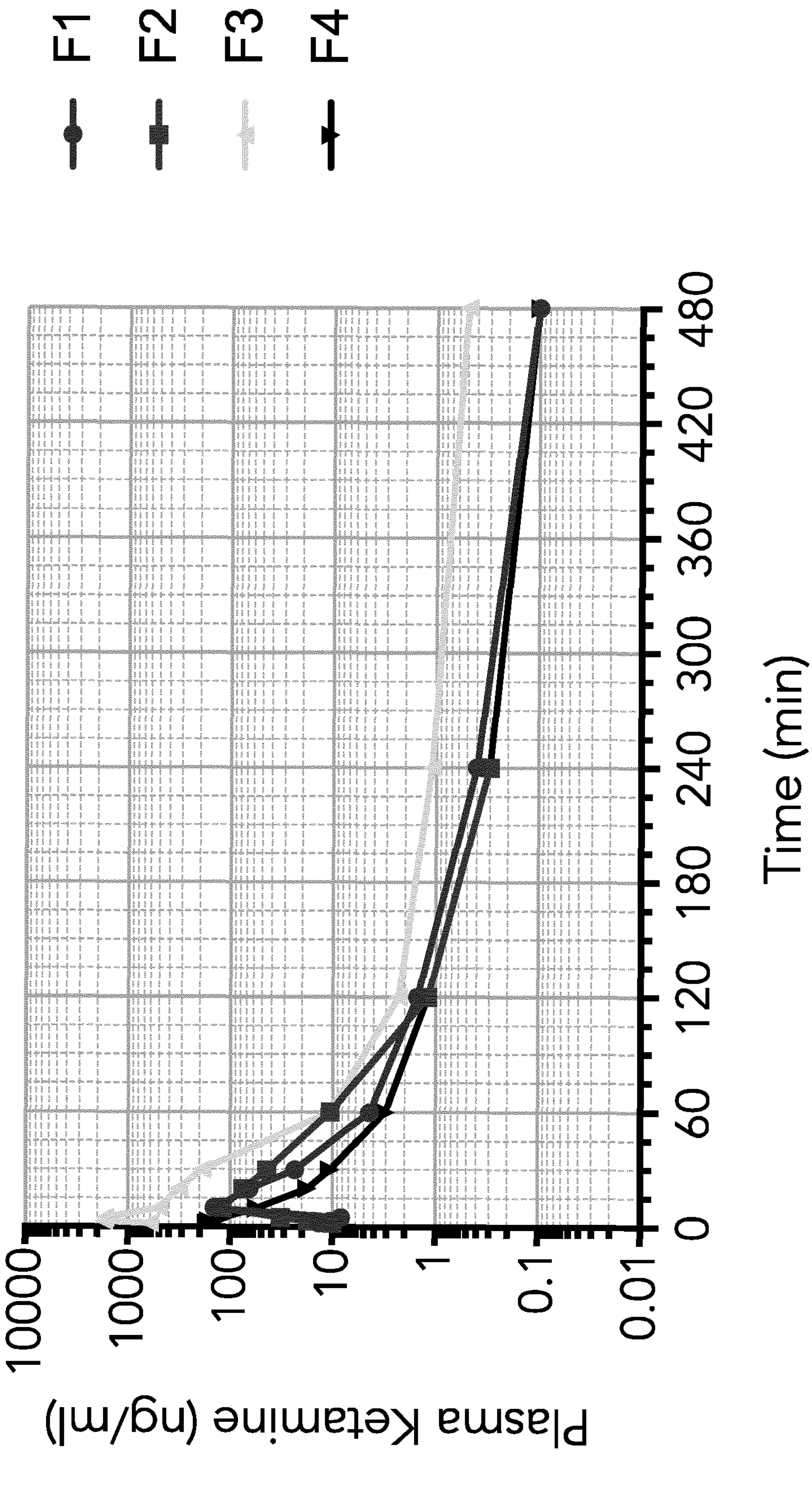
F1
F2
F3
F4
Plasma Ketamine (ng/ml)
10000
1000
100
10
1
0.1
0.01
Time (min)
0
60
120
180
240
300
360
420
480

Fig. 2

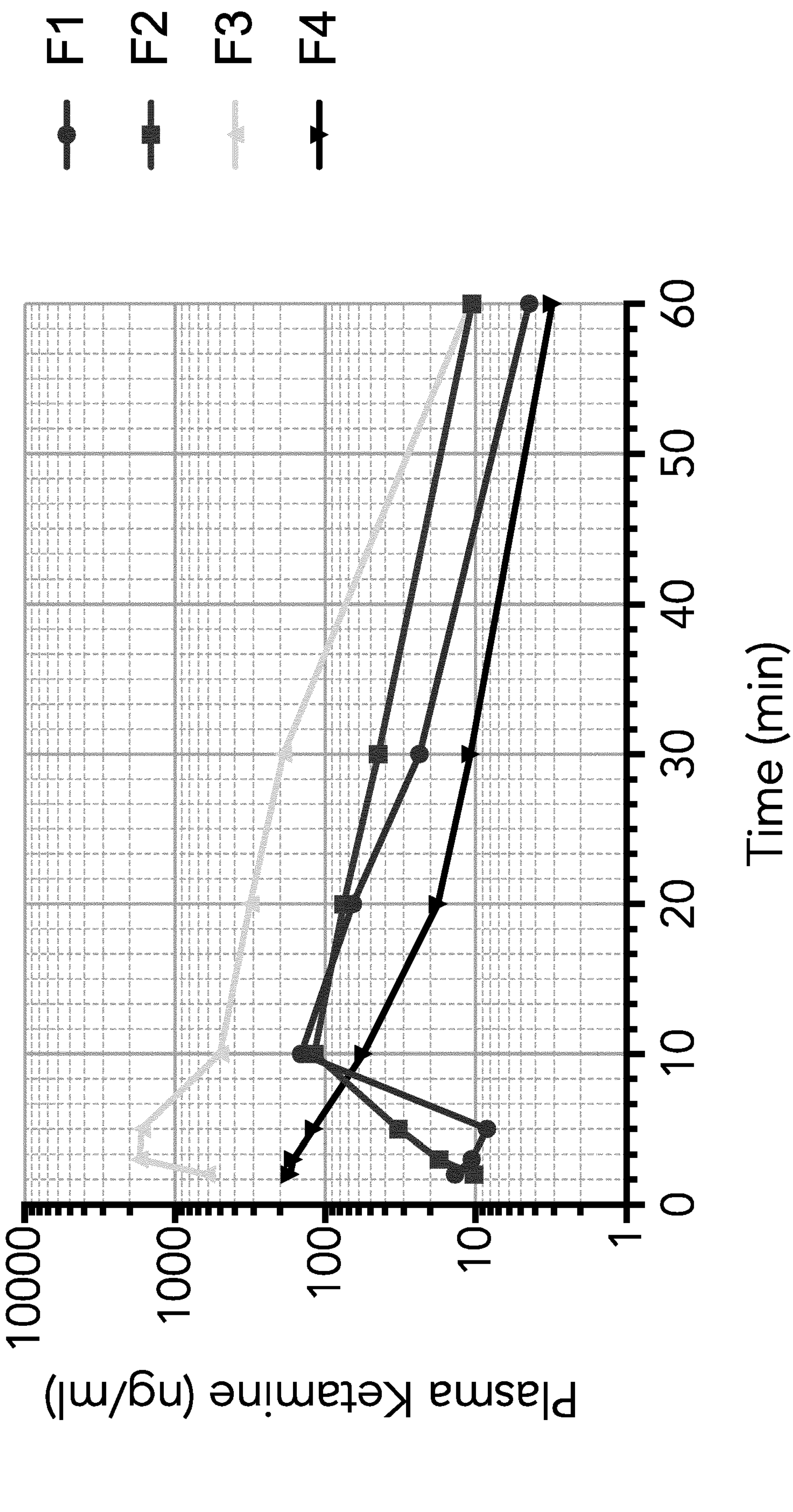
F1
F2
F3
F4
Plasma Ketamine (ng/ml)
10000
1000
100
10
1
0
10
20
30
40
50
60
Time (min)

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2015224070 A
- SE 2006050626 W


### Non-patent literature cited in the description


- **MULLER, J. ; PENTYALAM S. ; DILGER, J. ; PENTYALM S.** Ketamine enantiomers in the rapid and sustained antidepressant effects. *Ther Adv Psychopharmacol,* 2016, vol. 6, 185-192
- **HAAS, D. A. ; HARPER, D. G.** Ketamine: A Review of Its Pharmacologic Properties and Use in Ambulatory Anesthesia. *Anesth Prog,* 1992, vol. 39, 61-68
- Ketamine. **SINNER, B. ; GRAF, B.M.** Modern Anesthetics. Handbook of Experimental Pharmacology. 2008, vol. 182, 313-33
- **PRACHAYASITTIKUL, V. ; ISARANKURA-NA-AYUDHYA, C. ; TANTIMONGCOLWAT, T. ; NANTASENAMAT, C. ; GALLA, H.J.** EDTA-induced Membrane Fluidization and Destabilization: Biophysical Studies on Artificial Lipid Membranes. *Acta biochimica et biophysica Sinica,* 2007, vol. 39 (11), 901-913
- **MANAGARO, A. ; WERTZ, P.** *The effect of permeabilizer on the in vitro penetration of propranolol through porcine buccal epithelium*
- **DATE, A.A. ; DESAI, N. ; DIXIT, R. ; NAGARSENKER, M.** Self-nanoemulsifying Drug Delivery Systems: Formulation Insights, Applications and Advances. *Nanomedicine,* 2010, vol. 5 (10), 1595-1616
- **POUTON, C.W.** Formation of poorly water-soluble drugs for oral administration: Physicochemical and physiological issues and the lipid formulation classification system. *European Journal of Pharmaceutical Sciences,* 2006, vol. 29 (3-4), 278-287
- *European Pharmacopoeia,* vol. 8, 2565
- **MARTTIN, E. et al.** The effect of methylated b-cyclodextrins on the tight junctions of the rat nasal respiratory epithelium: Electron microscopic and confocal laser scanning microscopic visualization studies. *J Control Release,* 1999, vol. 57, 205-213
- **MERKUS, F. W. H. M. et al.** Cyclodextrins in nasal drug delivery. *Adv Drug Del Rev,* 1999, vol. 36, 41-57
- **SHANNON, R. D.** Revised effective ionic radii and systematic studies of interatomic distances in halides and chalcogenides. *Acta Cryst. A,* 1976, vol. 32 (5), 751-767